# FASCICULE DE BREVET EUROPEEN

(11) **EP 3 962 600 B1**
(45) Date de publication et mention de la délivrance du brevet: **19.11.2025**
(21) Numéro de dépôt: 20731913.8
(22) Date de dépôt: 30.04.2020
(51) Int. Cl.: A61P 17/00, A61K 8/04, A61K 8/06, A61K 8/73, A61K 8/81, A61Q 19/00, A61P 17/08, A61P 17/10, A61K 31/715, A61K 31/716, A61K 47/32, A61K 9/00, A61K 31/78, A61K 47/10, A61K 47/12, A61K 47/36

(54) **NOUVELLE FORMULATION TOPIQUE MODULANT LES VOIES DE LA NOCICEPTION**
NEUE TOPISCHE FORMULIERUNG, DIE NOCIPIRATIONSSIGNALWEGE MODULIERT
NOVEL TOPICAL FORMULATION THAT MODULATES NOCICEPTION PATHWAYS

(30) Priorité: 02.05.2019 FR 1904628
(43) Date de publication de la demande: 09.03.2022
(73) Titulaire: Pierre Fabre Dermo-Cosmétique, 81500 Lavaur (FR)
(72) Inventeur: BIDAN, Catherine, 31860 LABARTHE SUR LEZE (FR); BOURNET, Estelle, 82710 BRESSOLS (FR)
(74) Mandataire: Regimbeau
(86) Numéro de dépôt international: PCT/FR2020/050727
(87) Numéro de publication internationale: WO 2020/221979

(56) Documents cités:
- EP-A1- 0 835 647
- EP-A1- 3 269 354
- DE-A1- 102015 216 609
- US-A1- 2004 076 696
- US-A1- 2010 256 100
- STEEN K H ET AL: "Topical acetylsalicylic, salicylic acid and indomethacin suppress pain from experimental tissue acidosis in human skin", PAIN, ELSEVIER SCIENCE PUBLISHERS, AMSTERDAM, NL, vol. 62, no. 3, 1 September 1995 (1995-09-01), pages 339 - 347, XP002560409, ISSN: 0304-3959, [retrieved on 20000405], DOI: 10.1016/0304-3959(95)00011-G

## Description

### Domaine technique

L'invention concerne une nouvelle composition cosmétique ou dermatologique pour application topique, sous forme d'une dispersion comprenant une association de carbomère, de polysaccharide naturel et de copolymère modifié hydrophobiquement. Cette composition a la capacité de moduler les voies de la nociception, elle est également utile dans la protection et/ou le traitement de la peau qui présente des sensations d'inconfort et/ou de peau irritée, ou de peau atteinte par une dermatose inflammatoire.

### Technique antérieure

L'épiderme est un épithélium pluristratifié qui exerce une fonction barrière de protection contre son environnement et ses agressions. Parmi ses multiples fonctions physiologiques, l'épiderme possède celle de constituer une barrière à la fois biologique, physique et chimique contre l'invasion de l'organisme par les microorganismes. Cette propriété de barrière physique de l'épiderme est notamment liée à sa structure. Ainsi l'épiderme est conventionnellement divisé en une couche basale de kératinocytes constituant la couche germinative de l'épiderme, une couche dite épineuse constituée de plusieurs couches de cellules polyédriques et enfin, un ensemble de couches supérieures appelé couche cornée (ou stratum corneum), constituée de kératinocytes au stade terminal de leur différenciation appelés cornéocytes. Un dysfonctionnement de l'organisation structurelle cellulaire épidermique, ou un défaut de la fonction barrière chimique de l'épiderme, peut se traduire par un état inflammatoire cutané.

La peau est un organe dont l'innervation est très dense et va jusqu'aux couches les plus superficielles, à l'exception de la couche cornée. L'épiderme est également caractérisé par de nombreuses terminaisons nerveuses libres jusqu'aux couches les plus externes. Ces terminaisons nerveuses proviennent des neurones sensitifs issus des ganglions rachidiens situés le long de la moelle épinière. Ces neurones possèdent donc des axones extrêmement longs qui transmettent les informations sensitives : tactiles, chaleur mais également douleur, de la peau jusqu'au cerveau. La densité d'innervation varie en fonction de la zone du corps. Ainsi, chez les humains, les mains et le visage sont richement innervés par rapport au dos ou à l'abdomen.

On trouve dans la peau des fibres sensitives et des fibres nerveuses autonomes, toutes deux appartenant au système nerveux périphérique. Les terminaisons nerveuses de type sensitives répondent à une variété de stimuli physiologiques tels que la chaleur, le froid, le toucher, une distension mécanique et les UV. Certaines fibres peuvent même être activées par une variété de substances chimiques ou encore par des facteurs biologiques comme des agents microbiologiques ou des protéases provenant des plantes.

Tout individu a déjà fait l'expérience de la douleur. La douleur n'est pas une maladie, mais un avertissement. Une douleur connue et de courte durée est considérée comme normale. En effet, la douleur est un signal d'alarme qui informe le corps. Elle est définie par l'IASP (International Association for the Study of Pain), comme « une expérience sensorielle et émotionnelle désagréable, liée à une lésion tissulaire existante ou potentielle ou décrite en termes d'une telle lésion. Cette définition explique clairement que chacun vit sa propre expérience de la douleur. Cela peut aller de la douleur aiguë, comme celle provenant d'une aiguille ou d'une coupure ou encore de la douleur chronique due à un problème persistant, comme l'arthrite. Toutes les parties du corps bien évidemment peuvent faire l'objet de douleurs, notamment de douleurs aiguës, la peau n'est pas épargnée. La douleur au niveau du tissu cutané peut se présenter sous différentes formes : une sensation de brulure, de piqûre, de décharges électriques, de fourmillements, de démangeaisons, etc. Par ailleurs, certaines douleurs cutanées inexpliquées, plus récurrentes peuvent être dues à des neuropathies, on parle de neuropathies de petites fibres. Elles pourraient être responsables de douleurs inexpliquées se manifestant sous forme de picotement, tiraillement alors que la peau est normale : on parle d'allodynie.

L'allodynie est une douleur déclenchée par un stimulus qui est normalement indolore. Par exemple un léger effleurement de la peau ou une faible sensation de chaud ou froid peuvent alors être douloureux.

Les neurones qui répondent de façon sélective aux stimuli engendrés par les lésions tissulaires s'appellent des nocicepteurs. Ils sont constitués des fibres Aδ et C. Les fibres Aδ sont principalement des mécano-nocicepteurs qui interviennent dans les douleurs aiguës bien localisées. Les fibres C quant à elles sont des nocicepteurs polymodaux qui sont impliquées dans les douleurs sourdes, mal localisées.

Entre le message douloureux périphérique et la perception de la douleur, il existe ainsi une cascade électrique et chimique qui se divise en quatre étapes distinctes :
- la transduction, c'est le codage du message sensoriel pour l'acheminement de l'information nociceptive vers la corne postérieure de la moelle épinière. La transduction est réalisée par des récepteurs canaux qui dépolarisent la terminaison libre des nocicepteurs, activant ainsi des canaux sodiques dépendant du voltage (NaV1.6 - 1.9) permettant la génération et la propagation de potentiels d'action. Au niveau de la terminaison centrale du nocicepteur dans la corne postérieure de la moelle, les potentiels d'action vont provoquer l'entrée de calcium qui permettra la libération de nombreux neuromodulateurs et molécules de signalisation dans la fente synaptique. Il existe à ce niveau une modulation du signal nociceptif de type excitateur ou inhibiteur ;
- la transmission, le potentiel d'action nociceptif chemine dans le nocicepteur jusqu'à la corne dorsale de la moelle où il sera transmis à un neurone de second ordre spinothalamique. Le neurone secondaire croise immédiatement dans la moelle en passant sous le canal de l'épendyme pour former la voie spinothalamique en position ventrolatérale de la moelle et conduit l'information jusqu'à différentes régions des complexes ventrobasal et centromédian du thalamus somatosensoriel où il fera un contact synaptique avec le troisième neurone :
- la modulation, c'est l'ensemble des mécanismes par lesquels le message nerveux des nocicepteurs peut être modulé au niveau spinal mais aussi central. En particulier, il existe des contrôles inhibiteurs descendants issus du tronc cérébral qui s'exercent sur la transmission spinale des messages nociceptifs. Après stimulation nociceptive périphérique, il y a mise en jeu d'un contrôle inhibiteur diffus nociceptif provenant de nombreuses régions du tronc cérébral et mettant en jeu des voies noradrénergiques, sérotoninergiques et enképhalinergiques ; et
- la perception de la douleur est effective lorsque l'information nociceptive atteint le cerveau par les voies thalamocorticales. Le troisième neurone ou thalamo-cortical conduit ensuite les informations nociceptives vers différentes régions du cortex somatosensoriel et certaines structures limbiques. Les centres supérieurs de la douleur permettent de réaliser la complexité de l'équilibre entre les composantes sensorielles/discriminatives et affectives/émotionnelles de la douleur. L'activation du cortex préfrontal pourrait être en rapport avec les aspects cognitifs de la perception douloureuse.

La sensation de douleur cutanée est relativement fréquente, car environ une personne sur 3 serait concernée. Il est important de noter que 60% des patients qui se plaignent de douleur cutanée ont une dermatose. En outre, environ 50% des patients ayant une dermatose déclarent avoir une douleur cutanée, alors que seulement 10-15% des patients sans dermatose déclarent avoir une douleur cutanée. Il apparait que les principales dermatoses douloureuses sont la dermatite atopique, l'eczéma et le psoriasis, la rosacée, et la peau réactive, c'est-à-dire une peau fragile, intolérante. Les caractéristiques de la douleur ressentie chez les patients sont principalement des démangeaisons, une sensation de brûlure, des picotements.

Des millions de personnes sont concernées par ces douleurs cutanées, en France ce sont près de 4 millions de personnes qui se plaignent de douleurs cutanées et qui sont atteintes de dermatite atopique, de psoriasis ou d'eczéma de contact.

Les dermatoses inflammatoires sont des affections de la peau et des muqueuses, souvent douloureuses, qui se caractérisent par des manifestations inesthétiques comme des rougeurs et des plaques de desquamation. Plusieurs pathologies sont regroupées sous la dénomination de dermatoses inflammatoires. On peut citer à titre d'exemples non limitatifs : la dermatite atopique, l'eczéma, le psoriasis, la rosacée, le lichen plan, les prurigos, les dermites séborrhéiques ou encore l'acné. Ces dermatoses résultent bien souvent de phénomènes inflammatoires et de désordres immunitaires.

La dermatite atopique est la manifestation cutanée de l'atopie. C'est une dermatose inflammatoire chronique, survenant sur un terrain génétiquement déterminé. Elle touche 15 à 30% des enfants et 2 à 10% des adultes. Sa prévalence est en augmentation constante dans les pays industrialisés ; elle a doublé, voire triplé, au cours des trois dernières décennies et elle est maintenant considérée comme une préoccupation majeure de santé publique. La dermatite atopique est souvent associée à d'autres désordres atopiques, tels que la rhinite allergique et l'asthme, on parle de marche atopique. Cette affection apparaît le plus souvent au cours de la petite enfance et se caractérise par des éruptions répétées pendant plusieurs années. Elle évolue par poussées entrecoupées de rémissions spontanées. Les lésions se caractérisent par une sécheresse cutanée importante associée à des manifestations inflammatoires : éruptions érythémateuses papuleuses, vésiculeuses, squameuses et très prurigineuses. Sur le plan histologique, la dermatite atopique se caractérise, comme bien des dermatoses, par une infiltration de lymphocytes, monocytes et de polynucléaires éosinophiles autour de petits vaisseaux et des capillaires ; sur le plan biochimique, elle se caractérise par l'expression de cytokines telles que la Thymic Stromal Lymphopoietin (TSLP), protéine majeure dans le déclenchement de l'inflammation associée à la dermatite atopique. Par ailleurs, il a été montré que les chimiokines, notamment l'interleukine 8 (IL8) et les médiateurs lipidiques de l'inflammation tels que la prostaglandine 6KF1α, sont fortement impliqués dans les dermatoses telles que la dermatite atopique et dans les maladies inflammatoires chroniques en général.

L'eczéma est une dermatose prurigineuse caractérisée par une inflammation de la peau qui s'accompagne de rougeurs, de fines vésicules, de squames et de démangeaisons. Il peut commencer très tôt dans la vie, il s'observe même chez le nourrisson. Les personnes atteintes connaissent des périodes communément appelées « poussées d'eczéma », durant lesquelles les symptômes s'aggravent. Ces poussées, de durée variable, sont entrecoupées de périodes de rémission. L'eczéma serait un désordre de nature génétique, mais des facteurs environnementaux tels que la présence d'irritants chimiques ou le stress influenceraient son apparition.

Le psoriasis, maladie inflammatoire de la peau par excellence, se caractérise par l'apparition d'épaisses plaques de peau qui se desquament. Ces plaques sont présentes à différents endroits du corps, le plus souvent sur les coudes, les genoux et le cuir chevelu. Cette maladie chronique évolue par cycles, avec des périodes de rémission. Le psoriasis peut être très désagréable voire même douloureux lorsqu'il se manifeste sur la paume des mains, la plante des pieds ou dans les plis de la peau. Il existe plusieurs types de psoriasis, la forme la plus courante étant le psoriasis en plaques ou psoriasis vulgaire. Les autres formes sont le psoriasis en gouttes, érythrodermique et pustuleux.

La rosacée est une dermatose inflammatoire commune chronique et progressive liée à une relaxation vasculaire. Il s'agit d'une affection qui touche les petits vaisseaux du visage. Elle touche fréquemment les personnes à peau claire et peut avoir des conséquences psychoaffectives importantes. Le nom de cette pathologie fait référence à la couleur caractéristique que prend le visage lors de la maladie.

Le lichen plan est une éruption cutanée prurigineuse ne touchant que les adultes. Il s'agit d'une affection cutanée inflammatoire d'origine inconnue. Cette dermatose touche la peau, les muqueuses mais aussi les cheveux et les ongles.

Un prurigo est un prurit intense de la peau avec des papules érythémateuses et vésiculeuses avec lésions de grattage. Il s'agit le plus souvent d'une sensibilité exagérée aux piqures d'insectes, sensibilité qui se prolonge anormalement longtemps et qui est fréquente surtout chez le jeune enfant.

La dermite séborrhéique est une affection inflammatoire chronique de la peau, qui atteint les zones riches en glandes sébacées, à savoir le cuir chevelu et le visage. Elle est due à une levure, la Malassezia, présente dans la peau et qui se développe dans le sébum. Cette affection évolue par poussées favorisées par le stress, l'absence de soleil et la pollution.

L'acné est une pathologie cutanée commune, résultat d'une inflammation des follicules pilo-sébacés due en grande partie à la colonisation de *Cutibacterium acnes* dans l'infundibulum.

L'inflammation est une réaction de défense immunitaire normale de l'organisme à une agression de type : infectieuse, thermique, mécanique, chimique, lésionnelle ou encore allergique. Elle se caractérise en quatre points qui sont : la rougeur, la chaleur, le gonflement et la douleur.

L'inflammation cutanée aigüe est une réponse immédiate à un agent agresseur, de courte durée (quelques jours ou semaines), d'installation souvent brutale et caractérisée par un gonflement intense. Les inflammations aigües guérissent spontanément ou avec un traitement. La chronicité apparaît lorsque l'inflammation ne guérit pas spontanément, persiste ou s'aggrave pendant plusieurs mois voire plusieurs années. La réaction inflammatoire est un processus dynamique comportant plusieurs étapes successives : vasculaire (vasodilatation), la diapédèse des leucocytes et le chimiotactisme des cellules immunitaires et pour finir la détersion. La diapédèse leucocytaire correspond à la traversée active des parois vasculaires par les leucocytes et à l'accumulation de cellules immunitaires circulantes, les lymphocytes, les neutrophiles et les monocytes dans le foyer lésionnel. Les neutrophiles ont pour fonction d'attirer d'autres cellules inflammatoires par chimiotactisme et de nettoyer le site lésé en sécrétant des substances antimicrobiennes et des protéases. Les monocytes migrent par chimiotactisme et se différencient en macrophages nettoyant la zone lésée. Ils sécrètent des facteurs de croissance, des cytokines inflammatoires, comme IL-1, notamment IL-1β, TNFα, des protéases, des prostaglandines et des IFNs permettant le maintien et/ou l'amplification de l'inflammation. La détersion est consécutive à la phase vasculaire et contemporaine de la diapédèse leucocytaire. Il s'agit de l'élimination des tissus nécrosés et des agents pathogènes.

L'inflammation neurogène cutanée se définit comme l'induction et/ou l'amplification d'un processus inflammatoire primaire par les terminaisons nerveuses, ainsi il s'agit d'une inflammation de la peau induite par l'activation des fibres nerveuses intra-épidermiques qui sécrètent des neuropeptides tels que la substance P. L'inflammation neurogène cutanée est particulièrement impliquée dans les peaux irritées, les peaux présentant des sensations d'inconfort, voire dans les peaux atteintes de dermatoses inflammatoires, notamment prurigineuses. Le prurit est défini comme une sensation déplaisante qui provoque le besoin de se gratter. Il émerge depuis peu la notion de pruricepteurs, notamment spécifiques permettant de percevoir le prurit, mais leur distinction de la famille des récepteurs nociceptifs reste encore débattue. Ces pruricepteurs utilisent les fibres intra-épidermiques de type Aδ et surtout celles de type C. Ils sécrètent des neuropeptides : la substance P, le calcitonin gene related peptide (CGRP) et le vasoactive intestinal peptide (VIP). Depuis peu, le rôle des protéases (trypsine, cathepsine G, thrombine etc.) dans l'induction du prurit a été clairement établi. En effet, leur récepteur PAR-2 a été défini comme la deuxième grande voie d'activation du prurit (Misery et al., Nat. Rev. Neurol. 10, 408-416, 2014).

Les fibres nerveuses intradermiques interagissent directement ou indirectement avec les cellules cutanées et les cellules des systèmes endocrinien, lymphatique et immunitaire. Ces communications ont conduit à la définition d'un système neuro-immuno-endocrino-cutané. Pour fonctionner, ce système nécessite un langage commun constitué de molécules de différentes natures : les neuromédiateurs, les cytokines et des facteurs de croissance. Ces molécules sont synthétisées et libérées par les cellules de la peau, les cellules immunitaires résidentes et recrutées, ainsi que par les terminaisons nerveuses intra-épidermiques. Les cellules épidermiques et dermiques peuvent également produire des neuromédiateurs, des enzymes, des neurotrophines, des cytokines, des chémokines et des facteurs de croissance. Ces médiateurs régulent l'innervation cutanée et la réponse inflammatoire. En cas d'inflammation, les cellules immunitaires en transit ou présentes constitutivement dans la peau, peuvent s'activer sous l'action de ces médiateurs sécrétées par les terminaisons nerveuses sensorielles et les cellules de la peau. Ce processus conduit à une seconde vague de libération de cytokines et neuromédiateurs, qui engendrent une boucle d'amplification de l'inflammation.

Un nouveau concept émerge depuis peu, suggérant que les kératinocytes sont également des acteurs majeurs de l'inflammation neurogène cutanée.

A côté des dermatoses inflammatoires, les douleurs cutanées sont fréquentes chez des patients présentant une peau réactive, qui réagit à des stimuli qui sont sans effet pour d'autres types de peau. Cette hyper-réactivité de la peau résulte d'une diminution de son seuil de tolérance. Plus la peau est réactive, plus le seuil est bas.

La peau hyper-réactive ou peau très réactive est un état de la peau, désagréable à vivre par les personnes qui en sont affectées, mais très commun et qui peut provoquer des réactions cutanées visibles comme une peau sèche, des rougeurs, une desquamation. Mais elle peut provoquer également des réactions invisibles et par conséquent subjectives, décrites entre autres comme des sensations d'échauffements, de démangeaisons, de tiraillement ou de picotements.

Une peau douloureuse est définie comme une peau qui est le siège de sensations cutanées désagréables, à savoir des picotements, des démangeaisons, des sensations de brûlure, parfois en réaction à différentes agressions d'intensités diverses qui seraient bien tolérées par une peau normale. Une version plus extrême et fréquente d'une peau douloureuse est la peau hypersensible se caractérisant par des sensations désagréables d'inconfort et des réactions visibles lorsqu'elle est en contact avec des stimuli externes ou internes, qui seraient d'ordinaire inoffensifs, comme des températures extrêmes, des produits chimiques ou le rayonnement UV. Ces sensations et leur intensité est variable selon les personnes, ce qui rend le diagnostic d'autant plus difficile.

Trois signes sont communément observés chez les personnes ayant une peau douloureuse : une barrière cutanée altérée, des fibres sensorielles très réactives dans l'épiderme et des rougeurs.

La barrière cutanée altérée provoque également une déshydratation trans-épidermique qui rend la peau plus sensible à la pénétration de facteurs externes irritants.

Les fibres sensorielles de l'épiderme hyper-réactives ou sur-stimulées réagissent plus vite et de façon plus marquée que celles de la peau normale. Ces fibres sensorielles déclenchent des sensations cutanées désagréables qui sont décrites comme, entre autres, des sensations de picotement, d'échauffement ou de tiraillement de la peau du visage ou du cuir chevelu sans que l'on observe de signes visibles. Dans les cas les plus graves, les signes invisibles s'accompagnent de sécheresse cutanée, d'éruption cutanée ou de rougeurs.

En l'absence de sensations cutanées désagréables, la peau n'est pas considérée comme hypersensible. Toutefois, les rougeurs en elles-mêmes ne sont pas spécifiques à la peau hypersensible.

Les réactions sur une peau hypersensible peuvent être déclenchées par des facteurs environnementaux, psychologiques, externes et/ou mécaniques, tels que cités ci-après de façon non limitative. Les facteurs environnementaux incluent les brusques changements de température, la chaleur, le froid, le vent, le soleil et la pollution atmosphérique. Les facteurs psychologiques incluent les émotions fortes, le stress. Les facteurs externes incluent l'exposition à certains produits de nettoyage, la nourriture épicée, l'alcool, les parfums, les détergents, les piscines, l'eau excessivement calcaire, les bains et les douches. Les facteurs mécaniques impliquent une pression sur la peau.

La prévention de la douleur cutanée ou le traitement de cette dernière fait appel à plusieurs solutions. Il existe des compositions dermiques contenant des anesthésiques locaux tels que par exemple le butoforme, l'éthoforme ou d'autres produits à fonctions équivalentes. Cependant les anesthésiques de contact présentent souvent l'inconvénient d'entraîner une sensibilisation générale et non simplement locale comme désiré. En outre, ils sont fréquemment responsables de réactions allergiques dont les degrés de gravité peuvent passer de la simple dermite au choc anaphylactique.

Par ailleurs, les antalgiques ou analgésiques sont des médicaments destinés à réduire la douleur. Il existe 3 paliers définis selon leur activité. Le palier 1 concerne le paracétamol et les anti-inflammatoires non stéroïdiens, tels que l'aspirine, l'ibuprofène, la noramidopyrine qui sont prescrits en premier en cas de douleurs faible à modérée. Ils agissent principalement par inhibition de la cyclo-oxygénase. Le palier 2 concerne les antalgiques opiacés faibles comme la codéine, la dihydrocodéine, le tramadol. Ce type de substances agit au niveau du cerveau sur des récepteurs spécifiques responsables de l'abolissement de la douleur. Le palier 3 concerne les antalgiques opioïdes forts comme la morphine et ses dérivés. Ils sont utilisés en cas de douleurs intenses ou rebelles aux antalgiques de palier 2. Tous ces antalgiques possèdent des effets secondaires, allant des problèmes gastriques jusqu'à entrainer des problèmes de dépendances.

A côté de tous ces médicaments il existe des solutions plus douces, on peut citer certaines plantes et souches homéopathiques, comme l'arnica qui a démontré ses propriétés anti-inflammatoire et antalgique, notamment si elle est administrée dans les minutes suivant le choc.

Il existe également des sprays sans molécule thérapeutique, qui procurent un froid instantané qui anesthésie la douleur. Le froid permet la contraction des vaisseaux et empêche la diffusion du sang. Le froid par l'intermédiaire des mécanorécepteurs et/ou l'activation TRPM8, vient stimuler les interneurones pour moduler les voies de la nociception en tant que contre stimuli au niveau de la corne dorsale de la moelle épinière. Ces sprays peuvent contenir des gaz utilisés couramment en cryothérapie, comme par exemple le butane, l'isobutane, le propylène, le méthylal. Ils ont l'inconvénient de contenir des gaz propulseurs, qui sont inflammables et critiqués.

US 2004/076696 A1 décrit une composition à base d'hamamélis et de menthe poivrée exerçant un effet rafraîchissant destinée à soulager les brûlures, les démangeaisons et les picotements causés par la pince à épiler et le rasage, les démangeaisons de la peau et du cuir chevelu, les maux de tête, la fatigue, les coups de soleil, l'urticaire et les piqûres d'insectes.

EP 3 269 354 A1 décrit des compositions cosmétiques à base d'eau comprenant de l'agar, de la gomme de xanthane et un polymère hydrophile et procurant une sensation de fraîcheur au moment de l'utilisation.

L'article "Topical acetylsalicylic, salicylic acid and indomethacin suppress pain from experimental tissue acidosis in human skin" de Kay H. Steen et al (Pain, 62 (1995) 339-347) décrit une étude dans laquelle l'acide acétylsalicylique, l'acide salicylique et l'indométhacine ont été appliqués par voie topique et testés dans un modèle expérimental de douleur fournissant une excitation directe des nocicepteurs par le biais d'une acidose des tissus cutanés.

US 2010/256100 décrit un applicateur roll-on contenant une composition comprenant un niveau élevé d'acide salicylique destiné notamment au soin des peaux grasses et/ou acnéiques et/ou séborrhéiques.

Il existe un réel besoin de disposer de nouvelles compositions cosmétiques ou dermatologiques pour une utilisation topique apaisante, mais également utiles pour moduler les voies de la nociception, pour prévenir et/ou traiter l'inflammation neurogène cutanée, pour soulager toutes les douleurs cutanées, tout en évitant d'engendrer des effets secondaires, notamment ceux des anesthésiques locaux, antalgiques, analgésiques et des gaz procurant un froid instantané.

### Description détaillée de l'invention

La présente invention a pour objet de répondre à ces besoins. En effet, les inventeurs ont mis en évidence, de façon tout à fait inattendue, que l'association de 3 gélifiants présente des caractéristiques d'intérêt pour une utilisation topique apaisante. Cette association est également utile pour moduler les voies de la nociception, pour prévenir et/ou traiter l'inflammation neurogène cutanée, soulager des douleurs cutanées, et plus particulièrement des douleurs cutanées ressenties en présence de dermatoses inflammatoires. Ces effets sont obtenus notamment par la procuration d'un effet fraicheur lors de l'application d'une composition comprenant cette association des 3 gélifiants ; l'effet fraicheur contribue alors à diminuer la formation d'œdème et à la vasoconstriction des vaisseaux sanguins ce qui limite l'inflammation et entraîne un effet antalgique.

Un premier objet de l'invention concerne une composition cosmétique ou dermatologique comprenant une association de carbomère, de polysaccharide naturel, et de copolymère modifié hydrophobiquement ; ces trois composés peuvent être définis dans la suite de ce texte par le terme générique « gélifiants ».

La composition cosmétique ou dermatologique se présente sous la forme d'une émulsion comprenant une phase huileuse et une phase aqueuse. La phase aqueuse comprend l'association de carbomère, de polysaccharide naturel et de copolymère modifié hydrophobiquement.

Ainsi, la présente invention a pour objet une composition cosmétique ou dermatologique sous forme d'émulsion pour application topique, comprenant :
- une phase huileuse ; et
- une phase aqueuse comprenant un carbomère, un polysaccharide naturel et un copolymère acrylique ou méthacrylique réticulé modifié hydrophobiquement ;
la composition comprenant de 0,10 à 0,25%, de préférence de 0,05 à 0,20%, en poids de carbomère par rapport au poids total de la composition.

Sauf mention explicite contraire, le terme « un » désigne un ou plusieurs.

Ainsi, la phase aqueuse comprend un ou plusieurs carbomères, un ou plusieurs polysaccharides naturels et un ou plusieurs copolymères acryliques ou méthacryliques réticulés modifiés hydrophobiquement.

En d'autres termes, la présente invention a pour objet une composition cosmétique comprenant une dispersion d'une phase huileuse non gélifiée dans une phase aqueuse gélifiée, ladite phase aqueuse comprenant au moins l'association de carbomère, de polysaccharide naturel, et de copolymère modifié hydrophobiquement.

Les gélifiants sont des macromolécules d'origine naturelle ou synthétique capables de former des gels en emprisonnant une grande quantité de solvant, généralement de l'eau, dans un réseau en trois dimensions.

Le premier gélifiant est un carbomère. Les carbomères sont des polymères synthétiques hydrophiles d'acide acrylique, réticulé avec des éthers allyliques de pentaérythritol ou des éthers allyliques de saccharose. Les carbomères sont utilisés comme émulsifiants stabilisateurs ou comme agents épaississants ayant l'apparence d'un gel aqueux. On peut citer à titre d'exemple la matière commerciale Carbopol^{®}. Le numéro CAS de cette matière est : 9007-16-3.

Le deuxième gélifiant est un polysaccharide naturel. Les polysaccharides naturels sont constitués d'enchainements linéaires ou ramifiés d'unités saccharidiques, connectées par liaison glycosidique. Ces polysaccharides sont produits par une grande variété d'espèces qu'elles soient végétales, animales, bactériennes ou membres de la famille des champignons. Le polysaccharide naturel peut être choisi de façon non limitative parmi la gomme de scléroglucane, la gomme xanthane, la gomme guar, les pectines, les carraghénanes, les alginates, la gomme arabique, la gomme adragante, les amidons et la gélose. De façon préférée, le polysaccharide naturel est la gomme de scléroglucane. Celle-ci peut être obtenue par action fermentative du champignon filamenteux *Sclerotium rolfsii* sur un substrat glucosé. Le mélange est purifié avec de l'alcool (éthanol ou isopropanol), pressé, séché et moulu. La poudre ainsi obtenue peut éventuellement être stérilisée par irradiation ou par haute pression. La gomme de scléroglucane est un homopolysaccharide de masse moléculaire très élevée dont l'hydrolyse ne donne que des glucoses. La chaîne principale est constituée de β-D glucoses liés en β (1-3). Un glucose sur trois, porte un β-D glucose lié en β (1-6), empêchant l'agrégation des chaînes. La gomme de scléroglucane est également appelée gomme de sclérotium. Elle est disponible commercialement et est notamment commercialisée sous la dénomination ACTIGUM^{®} CS par la société SANOFI BIO INDUSTRIES, et en particulier ACTIGUM^{®} CS 11, et sous la dénomination AMIGEL^{®}, AMIGUM et AMIGEL^{®} GRANULE par la société ALBAN MULLER INTERNATIONAL, ou encore sous la dénomination Tinoderm^{®} SG-L par le groupe BASF. Cette gomme de scléroglucane peut éventuellement être modifiée, en particulier par traitements par des enzymes ou en utilisant des souches modifiées. Cependant, dans un mode de réalisation préféré, la gomme de scléroglucane n'est pas modifiée.

Le troisième gélifiant est un copolymère acrylique ou méthacrylique réticulé modifié hydrophobiquement, il s'agit notamment d'un copolymère d'acide acrylique ou méthacrylique et d'une longue chaîne d'alkyl acrylate réticulé avec par exemple un éther allyle de pentaérythritol ou de saccharose. La chaîne alkyle comprend avantageusement au moins 10 atomes de carbone, plus avantageusement 10 à 30 atomes de carbone. On peut citer à titre d'exemple le Pemulen^{®} TR-2. Les polymères Pemulen^{®} TR-1 et TR-2 possèdent au sein de leur structure de nombreux groupements hydrophiles, offrant des propriétés gélifiantes et des groupements alkyles présentant une affinité pour la phase grasse et responsables de leurs fonctions émulsionnantes. Cette double affinité permet la formation de microgels émulsionnés pour les lotions et les crèmes. La phase gélifiée est adsorbée autour de chaque gouttelette d'huile, offrant une stabilisation par répulsion physique.

La composition selon l'invention comprend le carbomère dans une teneur comprise entre 0,10 et 0,25%, en particulier entre 0,15 et 0,20% en poids relativement au poids total de la composition. Il est entendu que si la composition comprend plusieurs carbomères, la teneur totale en carbomères est comprise entre 0,10 et 0,25%, en particulier entre 0,15 et 0,20% en poids relativement au poids total de la composition.

La composition selon l'invention peut comprendre le polysaccharide naturel dans une teneur comprise entre 0,01 et 0,15%, en particulier entre 0,05 et 0,10% en poids relativement au poids total de la composition. Il est entendu que si la composition comprend plusieurs polysaccharides naturels, la teneur totale en polysaccharides naturels est comprise entre 0,01 et 0,15%, en particulier entre 0,05 et 0,10% en poids relativement au poids total de la composition.

La composition selon l'invention peut comprendre le copolymère modifié hydrophobiquement dans une teneur comprise entre 0,10 et 0,25%, en particulier entre 0,12 et 0,20% en poids relativement au poids total de la composition. Il est entendu que si la composition comprend plusieurs copolymères modifiés hydrophobiquement, la teneur totale en copolymères modifiés hydrophobiquement est comprise entre 0,10 et 0,25%, en particulier entre 0,12 et 0,20% en poids relativement au poids total de la composition.

Dans un mode de réalisation particulier, lorsque l'on exprime les quantités de carbomère, de polysaccharide naturel et de copolymère modifié hydrophobiquement dans l'association ternaire selon l'invention en parts, cette association comprend entre 1 et 5 parts de carbomère(s), entre 0,5 et 3 parts de polysaccharide(s) naturel(s) et entre 1 et 5 parts de copolymère(s) modifié(s) hydrophobiquement. De manière préférée, l'association selon l'invention comprend, en parts, 1 à 3 parts de carbomère(s), 0,5 à 2 parts de polysaccharide(s) naturel(s), et 1 à 3 parts de copolymère(s) modifié(s) hydrophobiquement. De façon encore préférée, l'association selon l'invention comprend, en parts, 2 parts de carbomère(s), 1 part de polysaccharide(s) naturel(s), et 2 parts de copolymère(s) modifié(s) hydrophobiquement.

De façon préférée, le ratio carbomère(s) / polysaccharide(s) naturel(s) / copolymère(s) modifié(s) hydrophobiquement est un rapport en poids respectivement de 2/1/2.

Dans certains modes de réalisation, la phase aqueuse comprend un seul carbomère, un seul polysaccharide naturel et un seul copolymère acrylique ou méthacrylique réticulé modifié hydrophobiquement.

La phase aqueuse d'une composition selon l'invention comprend de l'eau et éventuellement au moins un solvant hydrosoluble.

Par « solvant hydrosoluble », on désigne dans la présente invention un composé liquide à température ambiante et miscible à l'eau. Les solvants hydrosolubles utilisables dans la composition de l'invention peuvent en outre être volatils.

Parmi les solvants hydrosolubles pouvant être utilisés dans la composition selon l'invention, on peut citer notamment les monoalcools inférieurs ayant de 1 à 5 atomes de carbone tels que l'éthanol et l'isopropanol, les polyols, en particulier les glycols ayant de 2 à 8 atomes de carbone tels que l'éthylène glycol, le propylène glycol, le 1,3-butylène glycol et le dipropylène glycol, les cétones en C₃ et C₄ et les aldéhydes en C₂-C₄.

Selon un mode de réalisation de l'invention, la phase aqueuse d'une composition selon l'invention peut comprendre au moins un polyol en C₂-C₃₂, de préférence en C₃-C₁₆.

Par « polyol », il faut comprendre, au sens de la présente invention, toute molécule organique comportant au moins deux groupements hydroxyle libres.

De préférence, un polyol conforme à la présente invention est présent sous forme liquide à température ambiante.

Un polyol convenant à l'invention peut être un composé de type alkyle, linéaire, ramifié ou cyclique, saturé ou insaturé, portant sur la chaîne alkyle au moins deux fonctions -OH, en particulier au moins trois fonctions -OH, et plus particulièrement au moins quatre fonctions -OH.

Avantageusement, le polyol peut être choisi parmi l'éthylène glycol, le pentaérythritol, le triméthylolpropane, le propylène glycol, le 1,3 propanediol, le butylène glycol, l'isoprène glycol, le pentylène glycol, l'héxylène glycol, le glycérol, les polyglycérols, tels que les oligomères du glycérol comme le diglycérol, les polyéthylènes glycols, et leurs mélanges.

Selon un mode de réalisation préféré de l'invention, ledit polyol est choisi parmi l'éthylène glycol, le propylène glycol, le 1,3 propanediol, le butylène glycol, le glycérol, les polyglycérols, les polyéthylènes glycols, et leurs mélanges.

Selon un mode particulier, la composition selon l'invention peut comprendre au moins du propylène glycol.

Selon un autre mode particulier, la composition selon l'invention peut comprendre au moins du glycérol.

La phase aqueuse, qui comprend l'association des trois agents gélifiants, de l'eau et éventuellement au moins un solvant miscible à l'eau, peut être présente dans la composition en une teneur allant de 5% à 95%, de façon préférée de 30% à 90%, de façon encore préférée de 60% à 90% en poids, de façon encore plus préférée de 85% à 90% en poids, par rapport au poids total de ladite composition.

Au sens de l'invention, une phase huileuse est dispersée dans une phase aqueuse gélifiée. Elle inclut tous les corps gras liquides, généralement des huiles.

La phase huileuse est avantageusement non gélifiée.

Par phase huileuse non gélifiée, on entend que la phase huileuse ne comprend pas de gélifiants et/ou épaississant.

Au sens de l'invention, la phase huileuse est typiquement non volatile.

Par phase huileuse non volatile selon l'invention, on entend une variété d'huiles non volatiles convenant pour une composition cosmétique ou dermatologique. La phase huileuse non volatile peut être composée d'une large variété d'huiles synthétiques ou naturelles ou organiques, dont une liste non exhaustive est donnée à titre indicatif :
- les esters : des exemples d'huile non volatile utilisable selon l'invention comprennent les esters de formule RCO-OR' avec R et R', identiques ou différents, représentant une chaine, linéaire ou ramifiée, d'un alkyl, alkényl, alkoxycarbonylalkyl, ou alkoxycarbonyloxyalkyl ayant de 1 à 25 atmoes de carbone, de préférence de 4 à 20 atomes de carbone. Des exemples de tels esters incluent l'isononaote d'isotricédyle, le diheptanoate de PEG-4, le néopentanoate d'isostéaryle, le néopentanoate de tridécyle, l'octanoate de cétyle, le palmitate de cétyle, le ricinoléate de cétyle, le stéarate de cétyle, le myristate de cétyle, le dicaprylate/caprate de coco, l'isostéarate de décyle, l'oléate d'isodécyle, le néopentanoate d'isodécyle, le néopentanoate d'isohéxyle, le palmitate d'octyle, le malate de dioctyle, l'octanoate de tridécyle, le myristate de myristyle, l'octododécanol ;
- les esters glycérides d'acides gras : l'huile peut également comprendre les esters gras d'acides gras naturels, ou les triglycérides de source animale ou végétale. De tels exemples incluent, l'huile de castor, l'huile de lanoline, le citrate de triisocétyle, les triglycérides ayant de 10 à 18 atomes de carbone, les triglycérides capryliques/capriques, l'huile de noix de coco, l'huile de maïs, l'huile de coton, l'huile de lin, l'huile de vison, l'huile d'olive, l'huile de palme, le beurre d'illipé, l'huile de colza, l'huile soja, l'huile de tournesol, l'huile de noix et équivalent ;
- les glycérides d'acides gras : les huiles qui conviennent également sont les esters glycéryles synthétiques ou semi-synthétiques, comme les mono-, di-, triglycérides d'acides gras, qui sont des huiles ou des graisses naturelles modifiées, par exemple, le stéarate de glycéryle, le dioléate de glycéryle, le distéarate de glycéryle, le trioctanoate de glycéryle, le distéarate de glycéryl, le linoléate de glycéryle, le myristate de glycéryle, l'isostéarate de glycéryle, les huiles de castor PEG, les oléates de glycéryle PEG, les stéarates de glycéryle PEG, et équivalent ; et
- les hydrocarbones non volatils : ils conviennent également très bien à la composition selon l'invention en tant que phase huileuse non volatile, les hydrocarbones non volatils tels que les paraffines, les isoparaffines, les huiles minérales, et équivalent.

La phase huileuse non volatile préférée selon l'invention est un ester glycéride d'acide gras, notamment un triglycéride caprylique/caprique.

Avantageusement, la composition selon l'invention comprend entre 5 et 95% en poids, de façon préférée entre 10 et 70% en poids, de façon encore préférée entre 10 et 40% en poids, de façon encore plus préférée entre 10 et 15% en poids de phase huileuse par rapport au poids total de la composition.

Les compositions selon l'invention peuvent également comprendre en plus des actifs additionnels cosmétiques et dermatologiques. Les compositions selon l'invention peuvent comprendre des adjuvants cosmétiques choisis parmi les opacifiants, les stabilisants, les conservateurs, les polymères, les parfums, les agents épaississants ou agents gélifiants de la phase aqueuse différents du carbomère, de la gomme de sclerotium et du Pemulen^{®}, les filtres solaires, les actifs dermatologiques ou cosmétiques, les charges, les agents de suspension, les matières colorantes ou tout autre ingrédient habituellement utilisé en cosmétique pour ce type d'application. Il relève des opérations de routine de l'homme de l'art d'ajuster la nature et la quantité des additifs présents dans les compositions conformes à l'invention, de telle sorte que les propriétés cosmétiques désirées de celles-ci n'en soient pas affectées.

Comme exposé ci-avant, une composition selon l'invention peut être avantageusement cosmétique ou dermatologique.

Dans ce mode de réalisation particulier, une composition selon l'invention étant destinée à une application topique, elle contient un milieu physiologiquement acceptable.

Par « application topique », on entend une application sur la peau, les muqueuses et/ou les phanères.

Au sens de la présente invention, on entend par « milieu physiologiquement acceptable », un milieu compatible avec la peau, les muqueuses et/ou les phanères.

Ainsi le milieu physiologiquement acceptable est notamment un milieu cosmétiquement ou dermatologiquement acceptable.

Dans la présente invention, on entend désigner par « cosmétiquement ou dermatologiquement acceptable » ce qui est utile dans la préparation d'une composition cosmétique ou dermatologique, qui est généralement sûr, non toxique et ni biologiquement ni autrement non souhaitable et qui est acceptable pour une utilisation cosmétique ou dermatologique notamment par application topique.

Selon un mode particulier de l'invention, la composition cosmétique ou dermatologique est une composition thixotrope.

La définition de la thixotropie communément admise est la suivante : une composition est communément appelée thixotrope, si partant d'un état de repos pendant une période suffisamment longue, sa viscosité décroit avec le temps et sa structure se modifie, lorsqu'un gradient de cisaillement constant lui est appliqué. De manière réversible, si le cisaillement est interrompu, la viscosité croît de nouveau, la composition recouvre alors graduellement la consistance et la structure qu'elle avait au repos. Cet effet thixotrope est présenté dans l'exemple 3.

On associe souvent une sensation aqueuse à un effet frais rémanent apporté par la composition. On entend par composition apportant un effet frais rémanent, une composition qui, après application, induit une sensation de réduction de la température cutanée perceptible, cet effet frais durant de l'application jusqu'à au moins 5 minutes après application. En effet l'effet frais ressenti n'est pas mesurable, il s'agit d'une notion subjective. L'effet frais ressenti par l'application de la composition selon l'invention est clairement démontré dans l'exemple 4. Dans le cadre de la présente description, on utilisera de manière indifférente les termes frais, froid et fraîcheur.

La composition selon l'invention est plus particulièrement destinée au traitement de la peau et des muqueuses et peut se présenter sous forme d'onguents, de crèmes, de laits, de pommades, de poudres, de tampons imbibés, de solutions, de gels, de sprays, de mousses, de suspensions, de lotions, de sticks, de shampoings. Elle peut également se présenter sous forme de suspensions de microsphères ou nanosphères ou vésicules lipidiques ou polymériques ou de patchs polymériques et d'hydrogels permettant une libération contrôlée. Cette composition par voie topique peut se présenter sous forme anhydre, sous forme aqueuse ou sous la forme d'une émulsion.

La composition selon l'invention est administrée de préférence sous la forme d'une composition pulvérisable par spray.

De façon avantageuse, le spray fonctionne sans gaz propulseur.

Le spray peut être obtenu par des moyens conventionnels de formulation connus de l'homme du métier. Par exemple, la composition peut être pulvérisée par un pulvérisateur mécanique qui pompe la composition au sein d'un récipient, flacon ou équivalent. La composition passe à travers une buse qui peut être dirigée directement à l'endroit désiré de l'application. La buse peut être choisie de façon à appliquer la composition sous forme d'une vaporisation ou d'un jet de gouttelettes, selon les techniques de l'homme du métier. Un des avantages de l'application de la composition selon l'invention par spray est d'éviter l'application aux doigts qui peut être source de douleur par contact et/ou vecteur d'infections, d'autant plus lorsque la barrière cutanée est altérée.

La composition selon l'invention peut être pulvérisée de près, c'est-à-dire à quelques centimètres de la peau ou de plus loin, c'est-à-dire au-delà de 10 cm de la peau ; il est intéressant de noter que les effets recherchés sur la peau seront différents en fonction de la distance de pulvérisation par rapport à la peau.

Selon un mode de réalisation de l'invention, la composition pulvérisable selon l'invention sera pulvérisée de près, c'est-à-dire à moins de 10 cm de la peau, préférentiellement à 5-6 cm de la peau, et de façon encore préférée à 3-4 cm de la peau. Cette pulvérisation de près engendrera une couche gélifiée relativement solide sur la peau, de quelques cm² de surface, plus ou moins fine selon la quantité pulvérisée.

La composition selon l'invention par sa propriété de thixotropie une fois pulvérisée sur la peau, se regélifie progressivement et uniformément jusqu'à former une couche stable, formant un « effet seconde peau ».

Par « couche stable » on entend au sens de la présente invention, une couche qui reste sur la peau au-delà d'une minute, préférentiellement au-delà de 5 minutes et de façon encore préférée au-delà de 10 minutes.

Cette couche relativement solide permet de protéger la zone visée, en agissant localement par un effet froid, tout en modulant les voies de la nociception sur cette zone ainsi protégée. Cette administration très localisée est parfaitement adaptée pour soulager des douleurs cutanées aiguës, lors de coups, de piqures d'insectes par exemple, mais également en post acte médical, comme par exemple des petits actes chirurgicaux, comme des biopsies ou des exérèses cutanées. De façon préférée, l'administration est réalisée immédiatement après l'acte médical, le coup ou la piqure d'insecte par exemple.

Selon un autre mode de réalisation de l'invention, la composition pulvérisable selon l'invention sera pulvérisée à plus de 10 cm de la peau, de façon préférée à plus de 15 cm de la peau et de façon encore plus préférée à plus de 20 cm de la peau. La composition selon l'invention ainsi pulvérisée ne va pas former de couche solide mais reste en fines gouttelettes.

L'administration de la composition est réalisée par pulvérisation de la composition à plus de 5 cm de la peau pour ressentir l'effet froid, préférentiellement à plus de 10 cm, plus avantageusement à plus de 15 cm, et de façon encore préférée à environ 20 cm de la peau. Cette pulvérisation plus diffuse va également entrainer un effet froid mais sur une zone plus vaste. Cette administration plus diffuse est parfaitement adaptée à des douleurs cutanées plus chroniques, notamment lors de dermatoses inflammatoires, mais également à des douleurs touchant une zone de peau plus vaste.

Selon un mode de réalisation de l'invention, le mécanisme de pulvérisation doit être capable de délivrer toujours la même quantité de produit. Les mécanismes permettant de contrôler la quantité de composition à délivrer par le spray sont également connus de l'homme du métier.

Selon un mode de réalisation de l'invention, la composition cosmétique ou dermatologique selon l'invention comprend en outre un extrait de liane Uncaria Tomentosa.

La liane du Pérou ou Uncaria Tomentosa est une plante appartenant à la famille de Rubiaceae. C'est une liane grimpante jusqu'à 20-30 m de long et la tige principale atteint jusqu'à 25 cm de diamètre. Elle possède des branches quadrangulaires, généralement pubérulentes, des stipules largement ovales-triangulaires, finement et densément pubérulentes à l'extérieur.

Les feuilles sont glabres ou subglabres, opposées, leurs faces inférieures sont pubérulentes à pubescentes, le limbe est ovale à ovale-oblongue, avec la base arrondie et la marge entière ou crénelée dans la moitié supérieure. Les épines sont situées à la base des feuilles, fortement incurvées, tomenteuses sur les jeunes branches, glabres sur les plus anciennes. Ce sont ces épines qui aident la plante à grimper aux arbres, généralement de 20 à 40 mètres, d'où le nom de « griffes de chat ».

L'inflorescence porte des bractées réduites, à petites têtes de 12 à 20 mm de diamètre, des pédoncules de 1,5 à 4 cm de long. Les fleurs sont sessiles à calice tubulaire, avec les lobes obtus. Les fruits sessiles sont des capsules, 0,8 à 1,2 cm de long, pubescents à l'extérieur. Les graines ont deux ailes longues et sont étroites.

Il existe 34 espèces *d'Uncaria* aux propriétés médicinales.

*Uncaria tomentosa* est une plante sud-américaine qui a été largement utilisée par les peuples primitifs, en particulier du Pérou. Ce sont les racines ou les écorces de tiges qui sont utilisées sur le plan médicinal aussi bien par voie orale que topique. De très nombreux traitements font l'objet de l'utilisation de cette plante : rhumatismes, arthrite, ulcères gastriques, fièvre, asthme, infections urinaires, infections virales, allergies. Ces indications font appel à un processus inflammatoire et/ou à un désordre immunitaire. Sur le plan clinique il a été reconnu des effets bénéfiques de l'*Uncaria tomentosa* contre la leucémie, les tumeurs, les ulcères, les infections et l'arthrite.

Trois classes de constituants jouent un rôle important dans l'activité de *Uncaria tomentosa* : les alcaloïdes, les triterpènes polyhydroxylés et les polyphénols.

D'un point de vue thérapeutique, les constituants les plus importants dans l'écorce de la tige *d'Uncaria* sont les alcaloïdes indoliques, surtout ceux à structure pentacyclique (ptéropodine, 25% du total alcaloïde, isoptéropodine, spéciophylline, uncarine F, mitraphylline et isomitraphylline). Du point de vue quantitatif, les alcaloïdes tétracycliques les plus importants sont la rynchophylline et l'isorynchophylline.

Parmi les stérols, le b-sitostérol est majoritaire, suivi du stigmastérol et de campestérol. Plusieurs triterpènes ont été isolés de l'écorce et de la tige de *Uncaria tomentosa,* acide ursolique, acide oléanolique, acide uncarique, acide floridique, deux acides trihydroxy-ursenoiques, acide dioxo-ursenoique, plusieurs glycosides de l'acide quinovique, tomentosides A et B.

Pour les polyphénols, des tannins et des procyanidines ont été identifiées, ainsi que le kaempférol et le dihydrokaempférol. Parmi les flavonoïdes isolés il y a l'épicatéchine et les cinchonaines la et Ib. D'autres composés mis en évidence appartenant à la classe des polyphénols sont les suivants : les acides protocatechuique, vanillique, caféique, syringique, ellagique ainsi que des dérivés de l'acide p-coumarique.

Au niveau pharmacologique, deux activités principales sont bien décrites : une activité anti-inflammatoire et une activité antalgique. Pour le côté anti-inflammatoire, les décoctions de l'écorce *d'Uncaria* inhibent la production de TNF_{α} et dans une moindre mesure celle de PGE₂ (Goncalves et al., 2005, Phytochemistry, 66(1), 89). L'extrait aqueux de l'écorce *d'Uncaria tomentosa* présente une activité anti-inflammatoire qui serait due au moins en partie à un glycoside de l'acide quinovique. Cet extrait atténue l'inflammation intestinale chronique induite par l'indométacine chez les rats. Il inhibe de 65 à 85% l'activation du facteur NF_{□B} et il est actif dans l'arthrite, seul ou comme adjuvant des thérapeutiques traditionnelles pour traiter la douleur, les raideurs et les inflammations. En comparant un extrait aqueux et un extrait éthanolique 80% de l'écorce, il a été constaté que l'activité anti-inflammatoire était significativement plus importante pour l'extrait hydroalcoolique. La mitraphylline, alcaloïde oxindolique pentacyclique présente dans l'extrait chloroformique de l'écorce d'*Uncaria* a été évalué *in vivo* sur différentes cytokines qui jouent un rôle dans l'inflammation. Elle inhibe de 50% la libération des interleukines 1α, 1β, 17 et TNF_{-α} de la même façon que la dexaméthasone. La mitraphylline réduit de 40% la production d'IL-4 à la différence du corticoïde qui n'a pas d'activité (Rojas-Duran et al., 2012, J.

Ethnopharmacol. 143(3), 801-804). Une étude clinique réalisée pendant 52 semaines, sur 40 patients atteints de polyarthrite rhumatoïde, ayant reçu 3 fois 20 mg d'extrait aqueux d'*Uncaria tomentosa* par jour, contenant 73,5 % d'alcaloïdes pentacycliques, sans alcaloïde tétracyclique, en double aveugle contre placebo, a montré une amélioration significative de leurs symptômes (articulations douloureuses et raideur matinale). Cependant, il n'y a eu aucun effet du traitement sur les articulations gonflées et sur la perception du patient sur l'évolution de sa maladie.

Au niveau de la douleur, une fraction alcaloïdique d'*Uncaria tomentosa* a montré une activité antinociceptive in vivo après une administration intra-péritonéale (Jurgensen et al., 2005, Pharmacology, Biochemistry and Behaviour, 81 :466-477). Les principaux résultats sont une suppression de la douleur aigüe induite par la capsaicine et la réponse à l'acide acétique abdominale. Avec un test à la formaline, une inhibition de la douleur est trouvée en phase précoce et tardive. Un des mécanismes d'action présumé est l'activation des récepteurs 5HT-2. Les alcaloïdes geissoschizine methyl ether et isorhynchophylline ont montré une activité agoniste de 5-HT1A et antagoniste de 5HT2A/2C.

La plante est exclusivement issue de collecte organisée, réalisée au Pérou, il faut attendre 3 ans pour que la liane soit productive. La partie aérienne est récoltée plusieurs fois par an sur le même pied en laissant environ 50cm de tige pour le renouvellement de la plante. L'écorçage, qui a lieu sur le site de récolte est suivi par la mise en fagots des écorces d'1m environ. Le séchage est réalisé au soleil sur une dalle bétonnée ou sur une bâche plastique pendant 4 à 5 jours selon la saison.

Selon un mode de réalisation de l'invention, l'extrait d'*Uncaria tomentosa* est un extrait liquide, plus particulièrement en extrait hydrophile, plus particulièrement encore un extrait aqueux et encore plus particulièrement un extrait hydroalcoolique ou hydroglycolique.

Cet extrait pourra notamment être obtenu par extraction avec au moins un solvant hydrophile, préférentiellement avec au moins un solvant aqueux et encore plus préférentiellement avec au moins un solvant hydroalcoolique.

Un tel extrait pourra être utilisé en l'état (solution avec le solvant d'extraction) ou sous forme d'extrait concentré ou d'extrait sec après évaporation partielle ou totale du solvant d'extraction.

Selon un mode préféré de réalisation de l'invention, lorsque l'extrait est un extrait hydroalcoolique, l'alcool est évaporé et la solution aqueuse obtenue est stabilisée par ajout de propylène glycol.

Selon un mode de réalisation de l'invention, la composition cosmétique ou dermatologique selon l'invention comprend en outre un extrait d'avoine.

De façon préférée l'extrait d'avoine est obtenu à partir de plantules d'avoine, et préférentiellement tel que décrit dans la demande WO 2010/054879 ; tel que décrit dans cette même demande WO 2010/054879, un tel extrait d'avoine présente une activité anti-inflammatoire.

Par « plantule d'avoine », on entend au sens de la présente invention l'avoine avant épiaison, c'est-à-dire au stade après germination (environ 2 semaines à 2 mois après germination) durant le stade de la montaison jusqu'à l'épiaison non comprise. On appelle « montaison » la phase de croissance qui correspond à l'élongation de la tige et à la montée de l'épi en formation, avant floraison. Des métabolites secondaires sont décrits dans la demande WO 2010/054879 comme composants d'un extrait de plantule d'avoine : les flavonoïdes et les saponines de type avenacoside. Ledit extrait est caractérisé par la présence de 2 à 15% de flavonoïdes et 0,2 à 2% d'avenacosides A et B.

Le procédé de préparation de l'extrait d'avoine peut être comme suit :
- séchage et broyage des parties d'avoine, de préférence les plantules d'avoine,
- extraction en solvant organique choisi dans le groupe constitué des cétones, des esters, des alcools en C1 à C4 et des mélanges en toute proportion miscible de ces solvants, et,
- centrifugation ou filtration.

Avantageusement, le solvant organique du procédé est choisi dans le groupe constitué de l'acétone, la méthyl éthyl cétone, la méthylisobutyl cétone, l'acétate d'éthyle, un alcool en C1 à C4 et un mélange en toute proportion miscible de ces solvants.

Le marc obtenu par l'étape d'extraction est ensuite séparé de l'extrait par centrifugation ou filtration et la solution peut être plus ou moins concentrée jusqu'à l'obtention d'un extrait sec.

Selon un mode d'exécution de l'invention, un support peut être rajouté lors de l'étape de séchage dans des proportions massiques par rapport à la matière sèche extraite pouvant varier de 1 à 75%. Le support peut être un sucre comme la maltodextrine, le lactose, de la silice ou tout autre support cosmétologiquement ou dermatologiquement acceptable.

Dans un autre mode de réalisation de l'invention, la solution est concentrée de façon à obtenir un mout comprenant de 60 à 80% de matière sèche, et de préférence 70% de matière sèche, en poids par rapport au poids total du mout.

Selon un autre mode de réalisation, l'invention vise une composition cosmétique ou dermatologique pour application topique telle que décrite précédemment pour son utilisation apaisante, calmante et/ou anti-irritante.

Selon un autre mode de réalisation, l'invention vise une composition cosmétique ou dermatologique pour application topique telle que décrite précédemment pour son utilisation dans la prévention et/ou le traitement de l'inflammation neurogène cutanée.

De manière particulière, la prévention et/ou le traitement de l'inflammation neurogène cutanée comprend, ou consiste en la protection et/ou le traitement de la peau irritée ou de la peau fragile.

Selon un autre mode de réalisation, l'invention vise une composition cosmétique ou dermatologique pour application topique telle que décrite précédemment pour son utilisation dans la prévention et/ou le traitement de dermatoses inflammatoires choisies parmi la dermatite atopique, l'eczéma et le psoriasis.

La composition cosmétique ou dermatologique pour application topique telle que décrite précédemment peut être utile pour moduler les voies de la nociception.

Selon un autre mode de réalisation, l'invention vise une composition cosmétique ou dermatologique pour application topique telle que décrite précédemment pour une utilisation dans la prévention et/ou le traitement des sensations de démangeaisons et/ou des douleurs cutanées.

Selon un mode particulier de l'invention, les démangeaisons et/ou les douleurs cutanées sont dues à des dermatoses inflammatoires choisies parmi la dermatite atopique, l'eczéma et le psoriasis.

Selon un autre mode particulier de l'invention, les démangeaisons et/ou les douleurs cutanées sont dues à une piqûre, un coup, ou à un acte médical ou chirurgical, notamment une exérèse.

Les compositions selon l'invention sont utiles pour prévenir et/ou traiter les sensations de démangeaisons et/ou des douleurs cutanées, en particulier les démangeaisons et/ou les douleurs cutanées dues à une dermatose inflammatoire, une piqûre, un coup ou un acte médical ou chirurgical.

Les compositions selon l'invention sont utiles pour soulager, apaiser ou calmer une peau irritée et/ou une peau fragile et/ou une peau douloureuse.

De manière particulière, l'effet apaisant, calmant, ultra-calmant ou soulageant comprend ou consiste en une diminution des sensations d'inconfort cutané.

De manière particulière, la modulation des voies de la nociception, comprend ou consiste en une moindre sensation cutanée désagréable.

La composition cosmétique ou dermatologique pour application topique telle que décrite précédemment est utile pour la fabrication d'une composition pharmaceutique ou dermatologique destinée à apaiser, calmer et/ou limiter l'irritation de la peau.

La composition cosmétique ou dermatologique pour application topique telle que décrite précédemment est utile pour la fabrication d'une composition pharmaceutique ou dermatologique destinée à prévenir et/ou traiter l'inflammation neurogène cutanée.

La composition cosmétique ou dermatologique pour application topique telle que décrite précédemment est utile pour la fabrication d'une composition pharmaceutique ou dermatologique destinée à prévenir et/ou traiter des dermatoses inflammatoires choisies parmi la dermatite atopique, l'eczéma et le psoriasis.

La composition cosmétique ou dermatologique pour application topique telle que décrite précédemment est utile pour la fabrication d'une composition pharmaceutique ou dermatologique destinée à moduler les voies de la nociception.

La composition cosmétique ou dermatologique pour application topique telle que décrite précédemment est utile pour la fabrication d'une composition pharmaceutique ou dermatologique destinée à prévenir et/ou traiter des sensations de démangeaisons et/ou des douleurs cutanées, en particulier les démangeaisons et/ou les douleurs cutanées dues à une dermatose inflammatoire, une piqûre, un coup ou un acte médical ou chirurgical.

Une méthode pour apaiser, calmer et/ou limiter l'irritation de la peau, comprend l'administration à un patient en ayant besoin d'une quantité efficace d'une composition cosmétique ou dermatologique pour application topique telle que décrite précédemment.

Une méthode pour prévenir et/ou traiter l'inflammation neurogène cutanée, comprend l'administration à un patient en ayant besoin d'une quantité efficace d'une composition cosmétique ou dermatologique pour application topique telle que décrite précédemment.

Une méthode pour prévenir et/ou traiter des dermatoses inflammatoires choisies parmi la dermatite atopique, l'eczéma et le psoriasis, comprend l'administration à un patient en ayant besoin d'une quantité efficace d'une composition cosmétique ou dermatologique pour application topique telle que décrite précédemment.

Une méthode pour moduler les voies de la nociception, comprend l'administration à un patient en ayant besoin d'une quantité efficace d'une composition cosmétique ou dermatologique pour application topique telle que décrite précédemment.

Une méthode pour prévenir et/ou traiter des sensations de démangeaisons et/ou des douleurs cutanées, en particulier les démangeaisons et/ou les douleurs cutanées dues à une dermatose inflammatoire, une piqûre, un coup ou un acte médical ou chirurgical, comprend l'administration à un patient en ayant besoin d'une quantité efficace d'une composition cosmétique ou dermatologique pour application topique telle que décrite précédemment.

Ces compositions topiques peuvent être utilisées quotidiennement, une à plusieurs fois par jour, sans limitation de durée de traitement.

Un procédé cosmétique pour procurer une action topique apaisante ou calmante, consiste à appliquer sur la zone de la peau concernée une quantité efficace de la composition topique selon l'invention.

### Description des figures

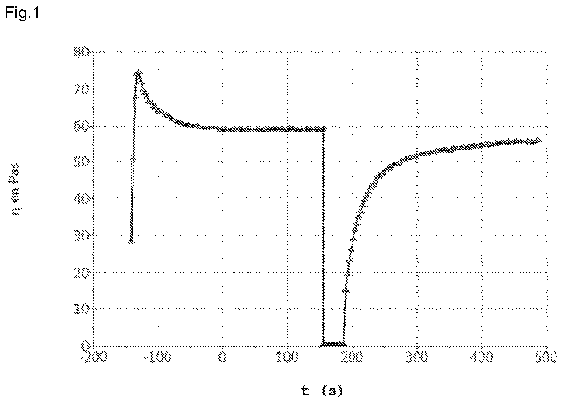

La figure 1 représente la mesure de la viscosité (η, en ordonnée, exprimée en Pa.s) de la composition selon l'invention au cours du temps (t, en abscisse, exprimé en secondes).

### Exemples

### Exemple 1 : exemple de composition selon l'invention

**Tableau 1**

| INCI désignation | Pourcentage en poids |
|---|---|
| Eau | QSP 100 % |
| Triglycéride caprylique/caprique | 2-20 % |
| Glycérine | 2-10 % |
| Polymère réticulé Acrylates/C10-30 Alkyl Acrylate | 0,10-0,25 % |
| Feuille d'avoine Avena Sativa/extrait de tige | 0,1-1 % |
| Acide benzoïque | 0,1-0,5 % |
| Caprylyl Glycol | 0,2-0,5 % |
| Carbomère | 0,10-0,25 % |
| Propylène Glycol | 0,1-10 % |
| Gomme de scléroglucane | 0,01-0,15 % |
| Hydroxyde de sodium | 0,1-0,5 % |
| Extrait d'Uncaria Tomentosa | 0,1-3 % |

### Exemple 2 : profil sensoriel de formules selon l'invention

L'objectif de ce test est de comparer les caractéristiques sensorielles de deux formules selon l'invention.

Les deux formules testées sont : la formule A correspond à celle de l'exemple 1, la formule B est la même mais sans extrait d'Uncaria Tomentosa.

### Méthodologie

Les conditions d'évaluation sont les suivantes :
A chaque début de séance, la température et l'hygrométrie de la pièce sont relevées, la température acceptable étant de 22°C ± 2°C. Chaque cabine est équipée d'un ordinateur, d'un lavabo avec débit d'eau régulier, d'une température d'eau et d'un éclairage standardisé.

Le test est réalisé par 15 femmes entraînées à la technique sensorielle pour les produits topiques ; en effet, ce sont des sujets experts ayant suivi et validé leur période de formation, selon la norme NF_ISO_8586:2012. Le panel d'experts analyse sensorielle est composé de volontaires avec un dossier médical à jour. Les sujets experts ne présentent pas de contre-indication médicale à leur participation à un test d'analyse sensorielle. Ils présentent tout type de peau saine. Il est demandé aux panélistes de venir en séance sans avoir appliqué de produits non rincés sur le visage et sur les avant-bras (maquillage, parfum, crèmes, lotions...), fumé, bu de café, utilisé de produits mentholés durant l'heure précédent le test.

Le produit sous forme de lait pour corps est appliqué sur les avant-bras, une formule par avant-bras. Les produits sont quantifiés au cours d'une même séance. Ils sont distribués à raison de 0,5 ml par application grâce à des seringues ou des pipettes automatiques adaptées à la quantité de produit nécessaire.

Descripteurs retenus pour l'étude :
Phase (1) à l'application
   - Facilité d'étalement : faculté du produit à se répandre sans résistance sur la peau
   - Vitesse de pénétration : rapidité du produit à pénétrer dans la peau
   - Fraîcheur : sensation de diminution de la température de la peau
   - Mouille : sensation d'humidité du produit entre les doigts
   - Huileux : comme de l'huile de cuisine entre les doigts
   - Collant : adhérence du doigt ou de la main sur la zone d'application
   - Crissant : accroche discontinue du produit entre les doigts
Phase (2) 5 minutes après application
   - Fraîcheur : sensation de diminution de la température de la peau
   - Tension : sensation de tiraillement
   - Brillance : aptitude de la peau à faire des reflets
   - Huileux : comme de l'huile de cuisine entre les doigts
   - Résistant : frein au passage de l'intérieur de la main sur la zone d'application
   - Collant : adhérence du doigt ou de la main sur la zone d'application
   - Filmogène : persistance d'un film sur la main
   - Toucher poudre : comme la peau de pêche (effet blur)
   - Souplesse : élasticité de la peau
   - Peau rebondie : épaisseur de la peau au pincement et au tapotement
   - Pelucheux : formation de petites particules sur la zone d'application.
Phase (3) 30 minutes après l'application

Idem sans la fraîcheur.

Les descripteurs ont été appris au cours de l'entrainement préalable. Ils sont expliqués oralement au début de chaque séance. Le questionnaire informatique (logiciel FIZZ Acquisition 2.51, Biosystemes) présente les échelles de mesures analogiques non structurées 0-100, pour chacun des descripteurs aux différentes phases sur l'écran d'ordinateur. Pour chaque descripteur, les intensités des 2 produits sont quantifiées sur 2 échelles, l'une correspondant au produit appliqué à gauche (formule A), l'autre au produit appliqué à droite (formule B).

A la fin de chaque phase, les sujets doivent indiquer via le questionnaire informatique s'ils ressentent des réactions indésirables comme des picotements, chaleur, rougeurs, démangeaisons, sensation de brûlure et tiraillement ou autres à préciser, en indiquer les intensités (peu intense, moyennement intense, intense et très intense) et leurs évolutions (augmentation, diminution, arrêt spontané pendant l'évaluation ou résolution après le rinçage normal des produits en fin de séance).

Les tests statistiques sont réalisés à l'aide du logiciel FIZZ traitement 2.60 (Biosystemes). L'analyse est faite descripteur par descripteur, avec une représentation des moyennes sous forme de radar et l'analyse de la significativité des différences entre les deux formules est réalisée par un test de Student.

Les résultats sont résumés dans le tableau 2.

Les inventeurs mettent ainsi clairement en évidence qu'une composition à base des 3 gélifiants selon l'invention présente un effet frais ressenti par des sujets expérimentés à ce genre de test sensoriel. Cet effet fraîcheur est même noté à 70 sur une échelle allant de 0 à 100, la note 0 correspondant à l'absence d'effet fraicheur et la note 100 correspondant à un effet fraicheur ressenti comme maximal.

Peu de différences sont mises en évidence entre ces deux formules. Pour l'ensemble des 15 sujets, les plus grandes différences ont porté sur les descripteurs indiqués dans le tableau 2 suivant :

**Tableau 2**

| | Formule A | | Formule B | | Différence | | |
|---|---|---|---|---|---|---|---|
| Attribut | Moy | SD | Moy | SD | Moy | SD | Stat |
| Fraîcheur 1 | 63,21 | 28,19 | 68,86 | 21,61 | 5,64 | 10,75 | 0,07 |
| Tension 2 | 10,79 | 23,0 | 15,36 | 24,34 | 4,57 | 7,67 | 0,04 |
| Brillance 2 | 29,71 | 28,44 | 38,07 | 29,07 | 8,36 | 17,09 | 0,09 |
| Peau Rebondie | 32,29 | 24,65 | 37,71 | 26,25 | 5,43 | 10,84 | 0,08 |

Dans les conditions de réalisation de cette étude, les caractéristiques sensorielles des deux formules sont très proches, 5 minutes après application la formule A laisse moins de sensations de tension, mais cette différence est faible. Les autres sensations n'ont pas été perçues significativement différentes.

Ainsi dans le contexte de cette étude, avec la comparaison entre les deux formules sur l'intensité de fraîcheur perçue, il est important de noter que la formule A qui contient un extrait de liane du Pérou, connu pour induire un effet frais ne se démarque pas de la formule B qui ne contient pas cet extrait. Cette sensation de fraîcheur est bien portée par l'association de carbomère, de polysaccharide naturel et de copolymère modifié hydrophobiquement.

### Exemple 3 : démonstration que la composition selon l'invention est thixotrope

La composition testée est celle de l'exemple 1.

La méthode consiste à mesurer la viscosité de la composition au cours du temps. La viscosité sert à mesurer la résistance d'un fluide à l'écoulement. Pour ce faire, un rhéomètre est utilisé ; il s'agit du rhéomètre Haake MARS60 de Thermofisher. L'étude a été réalisée à 22 °C, et c'est un cône plan de 1° qui a été utilisé comme mobile. Trois paliers sont effectués, le premier se fait avec une rotation à 0,1 s⁻¹ pendant 300 secondes, c'est-à-dire avec une contrainte vraiment faible, qui mime la viscosité au repos. Le second palier consiste en une rotation à 60 s⁻¹ pendant 30 secondes, (contrainte très forte) ce palier peut être assimilé au passage du produit en pompe. Enfin le troisième palier est réalisé avec une rotation de nouveau à 0,1 s⁻¹ pendant 300 secondes, ce qui permet de visualiser la réaction de la composition testée.

Les résultats sont présentés sur la Figure 1. Il en est déduit que la composition selon l'invention est thixotrope puisque sa viscosité remonte lentement après avoir été cisaillée. En effet, plus cette remontée est lente, plus l'effet thixotrope est marqué. Les inventeurs ont donc parfaitement démontré la thixotropie de la composition selon l'invention.

### Exemple 4: Etude clinique, efficacité à l'usage des peaux irritées chez l'adulte de la composition décrite dans l'exemple 1.

L'étude a été réalisée sur 33 patients, hommes ou femmes âgés de plus de 18 ans, présentant un phototype de I à IV. Tous ces patients présentent des irritations du visage et/ou du corps liées soit à des facteurs externes (frictions, détergents, climat) pour n=11 sujets, soit post-rasage pour n=11 sujets, soit à un érythème solaire pour n=11 sujets. Tous ces patients présentent des sensations d'inconfort liées à ces irritations perçues comme douloureuses avec un score de 3 sur une échelle de la douleur allant de 0 à 10 (le score 0 correspondant à une absence de douleur et le score 10 correspondant à la douleur maximale imaginable).

L'étude est menée sur 22 jours. Les consignes pour appliquer la composition selon l'invention sont de secouer et de vaporiser le produit à 20 cm de la zone irritée et de masser si nécessaire, et de l'utiliser jusqu'à 6 fois par jour.

Au cours du premier jour J1 de l'étude clinique, avant et après application, il y a une évaluation dermatologique :
- des signes physiques : érythème, œdème, desquamation, peau sèche, vésicule, papule, autres ;
- des signes fonctionnels liés à l'irritation cutanée : sensation de brûlure, sensation de chaleur, démangeaisons, tiraillement, picotements.

A côté de cette évaluation, il y a également un questionnaire de qualité de vie en dermatologie, une mesure de l'effet apaisant sur une échelle de 0 à 3 (0 correspondant à aucun effet apaisant ; 3 correspondant à un effet apaisant important) et une mesure de l'intensité des sensations d'inconfort perçues comme douloureuses sur une VAS (Visual Analog Scale, une échelle qui mesure un ressenti subjectif des sensations d'inconfort).

Toutes ces évaluations sont répétées à J3, J8 et J22 avec en plus un questionnaire d'acceptabilité (concernant un effet frais et un effet ultra-calmant).

Une première comparaison est faite pour chaque sujet et à chaque temps avant et après application, c'est l'effet immédiat. Une seconde comparaison est évaluée pour chaque sujet avant traitement, c'est-à-dire avant application du J1 versus après application de chaque temps, c'est l'effet cumulé. Un temps est défini par une application de la composition selon l'invention, quel que soit le jour d'application et quel que soit le nombre d'application réalisé par jour.

Il est à noter qu'après 21 jours d'utilisation sur les zones à l'étude et avec application de la composition selon l'invention de 1 à 6 fois par jour, aucun sujet n'a reporté de signe fonctionnel ou physique durant l'étude. Aucun sujet n'a présenté de nouveaux signes à J22 ni n'a présenté de signe aggravé par rapport à J1 (avant application). La tolérance de la composition selon l'invention est donc jugée comme excellente.

Les résultats des signes physiques sont représentés dans le tableau 3 ci-dessous :
Chaque sujet pour chaque visite reçoit un score de 0 à 4 (0 pour aucun signe, 1 pour très léger, 2 pour léger, 3 pour modéré et 4 pour sévère) lors d'une évaluation dermatologique de l'érythème, de la desquamation et de la sécheresse cutanée. Tous ces scores sont moyennés.

**Tableau 3**

| Jour | Erythème | Desquamation | Sécheresse |
|---|---|---|---|
| J1 T0 | 1,8 | 0,7 | 0,8 |
| J1 | 1,7 | 0,3 | 0,3 |
| J3 | 0,8 | 0,2 | 0,2 |
| J8 | 0,3 | 0,0 | 0,1 |
| J22 | 0,2 | 0,0 | 0,1 |

| | | | |
|---|---|---|---|
| J1 T0 : avant la première application de J1. | | | |

Ces résultats démontrent que la composition selon l'invention améliore les signes physiques de façon significative dès la première application et que cette amélioration s'intensifie au fur et à mesure de l'utilisation de la composition selon l'invention pendant les 3 semaines d'étude.

Les résultats des signes fonctionnels sont représentés dans le tableau 4 ci-dessous :
Chaque sujet pour chaque visite reçoit un score de 0 à 4 (0 pour aucun signe, 1 pour très léger, 2 pour léger, 3 pour modéré et 4 pour sévère) lors d'une évaluation dermatologique de sensations de brûlure, de démangeaisons, de picotements et de tiraillements. Tous ces scores sont moyennés.

**Tableau 4**

| Jour | Sensations Brûlure | Démangeaisons | Picotement | Tiraillement |
|---|---|---|---|---|
| J1 T0 | 1,0 | 1,2 | 0,6 | 0,2 |
| J1 | 0,4 | 0,5 | 0,2 | 0,1 |
| J3 | 0,1 | 0,2 | 0,0 | 0,0 |
| J8 | 0,0 | 0,0 | 0,0 | 0,0 |
| J22 | 0,0 | 0,0 | 0,0 | 0,0 |

| | | | | |
|---|---|---|---|---|
| J1 T0 : avant la première application de J1. | | | | |

Ces résultats démontrent que la composition selon l'invention améliore les signes fonctionnels de façon significative dès la première application et au fur et à mesure de l'utilisation de la composition selon l'invention pendant les 3 semaines d'étude.

Les résultats de l'évaluation de l'effet apaisant immédiat réalisée grâce au questionnaire de qualité de vie en dermatologie sont représentés dans le tableau 5 ci-dessous :

**Tableau 5**

| | J1T0 | J1 | J3T0 | J3 | J8T0 | J8 | J21T0 | J21 |
|---|---|---|---|---|---|---|---|---|
| Score (0-3) | 0 | 1,27 | 0,24 | 1,64 | 0,36 | 1,91 | 0,79 | 2,39 |

Les inventeurs mettent ainsi clairement en évidence une efficacité apaisante immédiate, avec une différence significative entre avant et après application de la composition selon l'invention.

L'effet de la composition selon l'invention sur la qualité de vie par diminution des sensations d'inconfort perçues comme douloureuses est également très positif. En effet, le questionnaire de qualité de vie en dermatologie a permis de démontrer qu'à J1 82% des sujets indiquent que les sensations d'inconfort ont un impact modéré à grand sur leur qualité de vie. A J3, il y a une diminution de l'impact sur la qualité de vie puisque 55% de sujets indiquent que les sensations d'inconfort ont un impact modéré à grand sur leur qualité de vie. A J8, une diminution encore plus marquée de l'impact sur la qualité de vie est constatée puisque seulement 33% des sujets indiquent que les sensations d'inconfort ont un impact modéré à grand sur leur qualité de vie. A J22, une grande majorité des sujets ont leur qualité de vie bien améliorée (82%). Les inventeurs montrent ainsi que la qualité de vie des sujets est améliorée au fur et à mesure de l'utilisation de la composition selon l'invention.

L'effet frais ressenti (noté de 0 à 10 dans le cadre du questionnaire d'acceptabilité de l'étude clinique) par les sujets (en %) lors de l'application de la composition selon l'invention est résumé dans le tableau 6 ci-dessous :

**Tableau 6**

| Score (0-10) | J3 | J8 | J22 |
|---|---|---|---|
| | Pourcentage de sujets | | |
| 0-4 | 3,0 % | 9,1 % | 9,1 % |
| 5 | 3,0 % | 3,0 % | 3,0 % |
| 6-7 | 15,2 % | 9,1 % | 0,0 % |
| 8-10 | 78,8 % | 78,8 % | 87,9 % |

L'effet frais est donc ressenti (score > 5) par environ 90% des patients au terme de cette étude.

L'effet immédiat ultra-calmant est également évalué dans le cadre du questionnaire d'acceptabilité de l'étude clinique et est résumé dans le tableau 7 ci-dessous (le score 0 correspondant à une absence d'effet immédiat ultra-calmant et le score 10 correspondant à un effet immédiat ultra-calmant important):

**Tableau 7**

| Score (0-10) | J3 | J8 | J22 |
|---|---|---|---|
| | Pourcentage de sujets | | |
| 0-4 | 9,1 % | 6,1 % | 6,1 % |
| 5 | 0,0 % | 3,0 % | 3,0 % |
| 6-7 | 21,2 % | 18,2 % | 12,1 % |
| 8-10 | 69,7 % | 72,7 % | 78,8 % |

Ainsi, l'effet immédiat ultra-calmant est ressenti (score > 5) par une majorité des sujets tout au long de l'étude, en effet c'est plus de 90% des sujets qui donnent un score d'au moins 6/10 pour juger cet effet.

En conclusion de cette étude clinique menée chez 33 sujets ayant des peaux irritées, les inventeurs démontrent l'efficacité de la composition selon l'invention sur tous les paramètres analysés.

### Exemple 5 : Etude clinique, efficacité à l'usage des peaux de patients atteints de psoriasis de la composition décrite dans l'exemple 1.

L'information sur l'étude a été fournie à chaque patient avant le début de l'étude. Ces informations étaient accessibles, compréhensibles et adaptées à chaque sujet d'essai. Il a été donné oralement puis dans un document écrit. Chaque patient a ainsi donné son consentement éclairé.

L'étude a été réalisée sur 30 patients, hommes ou femmes, âgés de 26 à 60 ans (51 ans de moyenne), présentant un phototype de I à IV. Tous ces patients présentent des irritations du visage et/ou du corps liées au psoriasis. Pour tous les patients, le psoriasis doit affecter moins de 10% de la surface corporelle et un PASI < 10 (Psoriasis Area Severity Index). Le but de cette étude est d'évaluer :
1/ la tolérance dermatologique de la composition selon l'invention et
2/ l'efficacité sur l'amélioration des signes physiques et fonctionnels.

L'étude est menée sur 21 jours ± 48 heures, sans interruption. Les consignes pour appliquer la composition quotidiennement selon l'invention sont de secouer et de vaporiser le produit à 20 cm de la zone irritée et de masser si nécessaire, et de l'utiliser de 1 à 6 fois par jour.

La tolérance de la composition selon l'invention est estimée par le dermatologue lui-même. Pour chaque sujet, l'évaluation dermatologique est réalisée lors des visites, c'est-à-dire au Jour 1 (J1, avant et 10 à 30 minutes après l'application), à la seconde visite au Jour 3 (J3), à la troisième visite au Jour 8 (J8 ± 1 jour) et à la visite finale après 21 jours d'application (J22 ± 2 jours).

Les signes suivants sont évalués :
- Signes physiques : érythème, œdème, desquamation, sécheresse de la peau, vésicules, papules, autres,
- Signes fonctionnels rapportés par le patient : sensation de brûlure, sensation de chaleur, démangeaisons, sensation de tiraillement, sensation de picotements, autres.

Pour chaque signe, l'intensité est reportée avec l'échelle suivante :
0 : absence de signe ; 1 : très léger ; 2 : léger ; 3 : modéré ; 4 : sévère ; puis la localisation, la durée et la fréquence.

Une estimation globale de la tolérance dermatologique est faite en tenant compte de tous les critères, elle se caractérise par 5 niveaux
- Mauvaise
- Modérée
- Bonne
- Très bonne
- Excellente (aucun signe physique ou fonctionnels reportés par les sujets).

L'évaluation de l'efficacité de la composition selon l'invention par l'amélioration de signes physiques et fonctionnels. Le dermatologue enregistre le score à J1 (avant et après application), à J3, J8 et J22 des signes fonctionnels reliés à l'irritation de la peau sur une échelle de 0 à 4 (0 : aucune irritation ; 1 : très légère ; 2 : légère ; 3 : modérée ; 4 sévère). Les signes physiques et fonctionnels sont les mêmes que ceux décrits pour l'évaluation de la tolérance.

### Résultats

Il est à noter qu'après 21 jours d'utilisation de la composition selon l'invention, appliquée de 1 à 6 fois par jour, aucun sujet n'a reporté de signe physique ou fonctionnel durant l'étude. Aucun sujet n'a présenté de nouveaux signes à J22 ni n'a présenté de signe aggravé par rapport à J1 (avant application). La tolérance de la composition selon l'invention est donc jugée comme excellente.

Les résultats des signes physiques sont représentés dans le tableau 8 ci-dessous.

**Tableau 8**

| | | Score de l'intensité ± esm | Statistiques vs J1To p |
|---|---|---|---|
| Erythème | J1 T0 | 2,45 ± 0,11 | - |
| | J1/ 10-30min | 2,36 ± 0,10 | 0,083 |
| | J3 | 1,94 ± 0,09 | <0,001 |
| | J8 | 1,61 ± 0,10 | <0,001 |
| | J22 | 1,32 ± 0,14 | <0,001 |
| Œdème | J1 T0 | 1,06 ± 0,06 | - |
| | J1/ 10-30min | 1,06 ± 0,06 | 1,000 |
| | J3 | 0,64 ± 0,13 | 0,034 |
| | J8 | 0,36 ± 0,13 | 0,004 |
| | J22 | 0,29 ± 0,13 | 0,002 |
| Sécheresse | J1 T0 | 1,79 ± 0,11 | - |
| | J1/ 10-30min | 0,56 ± 0,09 | <0,001 |
| | J3 | 0,91 ± 0,10 | <0,001 |
| | J8 | 0,81 ± 0,08 | <0,001 |
| | J22 | 0,11 ± 0,09 | <0,001 |
| Desquamation | J1 T0 | 1,73 ± 0,10 | - |
| | J1/ 10-30min | 0,64 ± 0,11 | <0,001 |
| | J3 | 0,81 ± 0,12 | <0,001 |
| | J8 | 0,45 ± 0,09 | <0,001 |
| | J22 | 0,29 ± 0,08 | <0,001 |
| Papule | J1 T0 | 2,00 | - |
| | J1/ 10-30min | 2,00 | - |
| | J3 | 0,00 | - |
| | J8 | 0,00 | - |
| | J22 | 0,00 | - |

La comparaison entre les différents temps se fait par une analyse statistique sur les rangs avec le test de Wilcoxon-Mann-Whitney. La significativité est atteinte lorsque p<0,05. esm : erreur standard à la moyenne.

Pour le critère de présence de papule, aucun test statistique n'est réalisé, trop peu d'effectif.

Pour les critères présence d'érythème ou d'œdème, une diminution statistiquement significative des scores moyennés est observée à partir de J3 et persiste à J22, comparé à J1 avant application de la composition selon l'invention. La réduction apparait plus marquée pour l'érythème.

Pour la sécheresse de la peau et la desquamation, des réductions très significatives (p<0,001) des scores moyennés sont observées dès la première application et persistent pendant toute la durée du traitement.

Les résultats des signes fonctionnels sont représentés dans le tableau 9 ci-dessous.

**Tableau 9**

| | | Score de l'intensité ± esm | Statistiques vs J1To p |
|---|---|---|---|
| Sensation de brûlure | J1 T0 | 2,00 ± 0,00 | - |
| | J1/ 10-30min | 0,67 ± 0,33 | 0,102 |
| | J3 | 0,33 ± 0,33 | 0,102 |
| | J8 | 0,33 ± 0,33 | 0,102 |
| | J22 | 1,00 ± 0,58 | 0,180 |
| Sensation de chaleur | J1 T0 | 2,00 | - |
| | J1/ 10-30min | 2,00 | - |
| | J3 | 1,00 | - |
| | J8 | 0,00 | - |
| | J22 | 0,00 | - |
| Démangeaison s | J1 T0 | 2,91 ± 0,08 | - |
| | J1/ 10-30min | 1,97 ± 0,14 | <0,001 |
| | J3 | 1,75 ± 0,15 | <0,001 |
| | J8 | 1,47 ± 0,12 | <0,001 |
| | J22 | 1,03 ± 0,14 | <0,001 |
| Sensation de tiraillement | J1 T0 | 2,33 ± 0,33 | - |
| | J1/ 10-30min | 0,67 ± 0,67 | 0,102 |
| | J3 | 0,67 ± 0,67 | 0,102 |
| | J8 | 0,33 ± 0,33 | 0,083 |
| | J22 | 0,33 ± 0,33 | 0,083 |
| Sensation de picotement | J1 T0 | 2,50 ±1 0,22 | - |
| | J1/ 10-30min | 1,83 ± 0,31 | 0,102 |
| | J3 | 1,17 ± 0,31 | 0,038 |
| | J8 | 1,17 ± 0,31 | 0,038 |
| | J22 | 0,83 ± 0,40 | 0,039 |

La comparaison entre les différents temps se fait par une analyse statistique sur les rangs avec le test de Wilcoxon-Mann-Whitney. La significativité est atteinte lorsque p<0,05. esm : erreur standard à la moyenne.

Pour le critère sensation de chaleur, aucun test statistique n'est réalisé, trop peu d'effectif.

Le score moyenné de la sensation de brûlure tend à diminuer après application de la composition selon l'invention, mais cette réduction n'atteint pas le seuil de significativité. Il en va de même avec la sensation de tiraillement.

La sensation de picotement est en revanche significativement atténuée dès J3 et persiste jusqu'à J22.

Les démangeaisons sont très fortement réduites par l'application de la composition selon l'invention, le score moyenné est statistiquement réduit (p<0,001) dès la première application et ce durant tout le traitement.

En conclusion, dans cette étude de 30 patients la composition selon l'invention est extrêmement bien tolérée chez les patients atteints de psoriasis. Elle est efficace en améliorant les signes physiques présents chez les patients (érythème, œdème, sécheresse et desquamation) et calme les démangeaisons. Son efficacité est rapide, dès 10 à 30 minutes après application et persiste pendant toute la durée du traitement.

### Exemple 6 : Etude clinique, efficacité à l'usage des peaux de patients atteints de dermatite atopique de la composition décrite dans l'exemple 1.

L'information sur l'étude a été fournie à chaque patient avant le début de l'étude. Ces informations étaient accessibles, compréhensibles et adaptées à chaque sujet d'essai. Il a été donné oralement puis dans un document écrit. Chaque patient a ainsi donné son consentement éclairé.

L'étude a été réalisée sur 33 patients, hommes ou femmes, âgés de 22 à 61 ans (43 ans de moyenne), présentant un phototype de I à IV. Tous les sujets présentent une dermatite atopique selon la définition de « The UK Working Party's Diagnostic Criteria for Atopic Dermatitis ». Ils présentent un SCORAD de 15 à 25, ils ont comme seul traitement un émollient qu'ils continuent à appliquer une fois par jour. Les sujets ont une sensation d'inconfort cutané, perçu comme douloureux et lié à la dermatite atopique. Pour être inclus, la sensation douloureuse doit atteindre au minimum le score de 3 sur une échelle visuelle de 0 à 10.

Le but de cette étude est d'évaluer comme dans l'exemple 5 :
1/ la tolérance dermatologique de la composition selon l'invention et
2/ l'efficacité sur l'amélioration des signes physiques et fonctionnels.

L'étude est menée sur 21 jours ± 48 heures, sans interruption. Les consignes pour appliquer la composition quotidiennement selon l'invention sont de secouer et de vaporiser le produit à 20 cm de la zone irritée et de masser si nécessaire, et de l'utiliser de 1 à 6 fois par jour.

Le protocole d'administration, les critères de tolérance et d'efficacité sont les mêmes que ceux décrits dans l'exemple 5.

Il est à noter qu'après 21 jours d'utilisation de la composition selon l'invention, appliquée de 1 à 6 fois par jour, quelques réactions sont apparues, mais la majorité d'entre elles étaient fugaces et avaient une intensité jugée légère. En outre, les réactions se sont généralement produites dans les premiers jours et ont été résolus après la première semaine d'utilisation. Si des signes d'inconfort ont été signalés, un seul signe physique a été reporté. La tolérance de la composition selon l'invention est donc jugée comme bonne, bien que la composition selon l'invention soit testée sur un panel de sujets atteints de dermatite atopique donc ayant la peau sensible et irritée.

Les résultats des signes physiques sont représentés dans le tableau 10 ci-dessous.

**Tableau 10**

| | | Score de l'intensité ± esm | Statistiques vs J1To p |
|---|---|---|---|
| Erythème | J1 T0 | 0,6 ± 0,1 | - |
| | J1/ 10-30min | 0,5 ± 0,1 | 0,500 |
| | J3 | 0,4 ± 0,1 | 0,0156 |
| | J8 | 0,3 ± 0,1 | 0,0156 |
| | J22 | 0,3 ± 0,1 | 0,0078 |
| Œdème | J1 T0 | 0,0 ± 0,0 | - |
| | J1/ 10-30min | 0,0 ± 0,0 | ND |
| | J3 | 0,0 ± 0,0 | ND |
| | J8 | 0,0 ± 0,0 | ND |
| | J22 | 0,0 ± 0,0 | ND |
| Sécheresse | J1 T0 | 2,5 ± 0,1 | - |
| | J1/ 10-30min | 1,3 ± 0,1 | <0,001 |
| | J3 | 1,0 ± 0,1 | <0,001 |
| | J8 | 0,7 ± 0,1 | <0,001 |
| | J22 | 0,2 ± 0,1 | <0,001 |
| Desquamatio n | J1 T0 | 2,1 ± 0,1 | - |
| | J1/ 10-30min | 0,6 ± 0,1 | <0,001 |
| | J3 | 0,3 ± 0,1 | <0,001 |
| | J8 | 0,1 ± 0,0 | <0,001 |
| | J22 | 0,0 ± 0,0 | <0,001 |
| Papule | J1 T0 | 0,5 ± 0,1 | - |
| | J1/ 10-30min | 0,4 ± 0,1 | 1,000 |
| | J3 | 0,3 ± 0,1 | 0,125 |
| | J8 | 0,2 ± 0,1 | 0,0313 |
| | J22 | 0,1 ± 0,1 | 0,0156 |

La comparaison entre les différents temps se fait par une analyse statistique sur les rangs avec le test de Wilcoxon-Mann-Whitney. La significativité est atteinte lorsque p<0,05. esm : erreur standard à la moyenne.

### Pour la présence d'œdème, aucun test statistique n'est réalisé, trop peu d'effectif.

Immédiatement après l'application de la composition selon l'invention, des diminutions statistiquement significative de la sécheresse cutanée et de la desquamation sont observées chez pratiquement tous les patients. Après 3 jours d'application, une diminution de l'érythème de 35% est observée chez un patient sur 2, une diminution de 88% de la desquamation est retrouvée chez tous les patients et une moindre sécheresse de la peau est également est notée. Ces résultats perdurent pendant tout le traitement et sont même encore améliorés puisque l'ensemble des signes physiques est diminué de 80% pour l'ensemble des patients.

Les résultats des signes fonctionnels sont représentés dans le tableau 11 ci-dessous.

**Tableau 11**

| | | Score de l'intensité ± esm | Statistiques vs J1To p |
|---|---|---|---|
| Sensation de brûlure | J1 T0 | 0,7 ± 0,2 | - |
| | J1/ 10-30min | 0,5 ± 0,2 | 0,3125 |
| | J3 | 0,2 ± 0,1 | 0,001 |
| | J8 | 0,1 ± 0,1 | <0,001 |
| | J22 | 0,0 ± 0,0 | <0,001 |
| Sensation de chaleur | J1 T0 | 0,4 ± 0,1 | - |
| | J1/ 10-30min | 0,3 ± 0,1 | 0,500 |
| | J3 | 0,0 ± 0,0 | 0,0313 |
| | J8 | 0,0 ± 0,0 | 0,0625 |
| | J22 | 0,0 ± 0,0 | 0,0625 |
| Démangeaison s | J1 T0 | 1,6 ± 0,2 | - |
| | J1/ 10-30min | 0,8 ± 0,1 | <0,001 |
| | J3 | 0,0 ± 0,0 | <0,001 |
| | J8 | 0,0 ± 0,0 | <0,001 |
| | J22 | 0,0 ± 0,0 | <0,001 |
| Sensation de tiraillement | J1 T0 | 1,7 ± 0,2 | - |
| | J1/ 10-30min | 0,6 ± 0,1 | <0,001 |
| | J3 | 0,0 ± 0,0 | <0,001 |
| | J8 | 0,0 ± 0,0 | <0,001 |
| | J22 | 0,0 ± 0,0 | <0,001 |
| Sensation de picotement | J1 T0 | 0,5 ± 0,2 | - |
| | J1/ 10-30min | 0,2 ± 0,1 | 0,0078 |
| | J3 | 0,1 ± 0,0 | 0,0020 |
| | J8 | 0,0 ± 0,0 | 0,0020 |
| | J22 | 0,0 ± 0,0 | 0,0039 |

La comparaison entre les différents temps se fait par une analyse statistique sur les rangs avec le test de Wilcoxon-Mann-Whitney. La significativité est atteinte lorsque p<0,05. esm : erreur standard à la moyenne.

Immédiatement après l'application de la composition selon l'invention, une diminution statistiquement significative des démangeaisons est notée chez presque tous les patients. De même il est observé des réductions marquées et significatives des sensations de tiraillement et de picotement chez presque l'ensemble des personnes testées. Après 3 jours de traitement, l'ensemble des signes fonctionnels sont significativement réduits, par exemple une réduction de 98% des démangeaisons est répertoriée. Tous ces signes sont significativement diminués pendant toute la durée du traitement, même si avec la sensation de chaleur le seuil de significativité n'est pas tout à fait atteint (p=0.06). Après 22 jours de traitement, les démangeaisons, les sensations de tiraillement et de picotement sont même complètement absentes chez tous les patients montrant l'efficacité de la composition selon l'invention.

En conclusion, dans cette étude de 33 patients la composition selon l'invention est bien tolérée chez les patients atteints de dermatite atopique. Elle est efficace en améliorant les signes physiques et fonctionnels des patients. Son efficacité est rapide, dès 10 à 30 minutes après application et persiste pendant toute la durée du traitement.

### Exemple 7 : Etude clinique, efficacité à l'usage des peaux de patients ayant une sensation de douleur aux mains dues à des irritations, de la composition décrite dans l'exemple 1.

L'information sur l'étude a été fournie à chaque patient avant le début de l'étude. Ces informations étaient accessibles, compréhensibles et adaptées à chaque sujet d'essai. Il a été donné oralement puis dans un document écrit. Chaque patient a ainsi donné son consentement éclairé.

L'étude a été réalisée sur 16 patients, hommes ou femmes, âgés de 26 à 58 ans (47 ans de moyenne), présentant un phototype de III à IV. Tous les sujets présentent au niveau des mains de l'eczéma qui engendre une sensation d'inconfort cutané perçu comme douloureux. Les patients n'avaient aucun traitement au moment de l'inclusion. Pour être inclus, chaque patient doit avoir une sensation douloureuse qui atteint au minimum le score de 3 sur une échelle analogique visuelle de 0 à 10.

Le protocole expérimental est le même que celui des exemples 5 et 6.

La tolérance dermatologique de la composition selon l'invention est testée, les critères de tolérance sont les mêmes que ceux décrits dans l'exemple 5.

L'efficacité apaisante de la composition selon l'invention est évaluée sur une échelle de 0 à 3 (0 : aucune ; 1 : modérée ; 2 : bonne ; 3 : très bonne). Sa capacité à diminuer l'intensité de l'inconfort cutané perçu comme pénible est mesurée grâce à une échelle analogique visuelle de 0 à 10.

### Résultats

Il est à noter qu'après 21 jours d'utilisation de la composition selon l'invention, appliquée de 1 à 6 fois par jour, aucun sujet n'a reporté de signe physique ou fonctionnel durant l'étude. Aucun sujet n'a présenté de nouveaux signes à J22 ni n'a présenté de signe aggravé par rapport à J1 (avant application). La tolérance de la composition selon l'invention est donc jugée comme excellente malgré l'application sur une peau très irritée.

L'efficacité apaisante est résumée dans le tableau 12 ci-dessous.

**Tableau 12**

| Cinétique | Score moyenné ± esm | Statistique Valeur p |
|---|---|---|
| J1-J1T0 | 1,38 ± 0,13 | <0,001 |
| J2-J1T0 | 1,44 ± 0,13 | <0,001 |
| J3-J1T0 | 1,44 ± 0,13 | <0,001 |
| J4-J1T0 | 1,50 ± 0,13 | <0,001 |
| J5-J1T0 | 1,69 ± 0,18 | <0,001 |
| J6-J1T0 | 1,63 ± 0,15 | <0,001 |
| J7-J1T0 | 1,69 ± 0,15 | <0,001 |
| J8-J1T0 | 1,81 ± 0,16 | <0,001 |
| J9-J1T0 | 1,88 ± 0,18 | <0,001 |
| J10-J1T0 | 1,94 ± 0,17 | <0,001 |
| J11-J1T0 | 2,00 ± 0,18 | <0,001 |
| J12-J1T0 | 2,00 ± 0,18 | <0,001 |
| J13-J1T0 | 2,00 ± 0,18 | <0,001 |
| J14-J1T0 | 2,00 ± 0,18 | <0,001 |
| J15-J1T0 | 2,00 ± 0,18 | <0,001 |
| J16-J1T0 | 2,00 ± 0,18 | <0,001 |
| J17-J1T0 | 2,06 ± 0,17 | <0,001 |
| J18-J1T0 | 2,25 ± 0,19 | <0,001 |
| J19-J1T0 | 2,25 ± 0,19 | <0,001 |
| J20-J1T0 | 2,25 ± 0,19 | <0,001 |
| J21-J1T0 | 2,25 ± 0,19 | <0,001 |

La comparaison entre les différents temps (par rapport J1T0, avant application) se fait par une analyse statistique sur les rangs avec le test de Wilcoxon-Mann-Whitney. La significativité est atteinte lorsque p<0,05. esm : erreur standard à la moyenne.

Sous ces conditions expérimentales, après application 1 à 6 fois par jour de la composition selon l'invention, une efficacité immédiate apaisante significative dès une première utilisation du produit est observée à J1 et de manière continue jusqu'à 21 jours d'application.

Les résultats sur l'effet immédiat de l'application de la composition selon l'invention sont présentés dans le tableau 13 ci-dessous.

**Tableau 13**

| Cinétique | Score moyenné ± esm | Statistique Valeur p | % de sujets avec une amélioration |
|---|---|---|---|
| J1imm - J1T0 | -1,38 ± 0,26 | <0,001 | 81 |
| J2imm - J2T0 | -1,00 ± 0,30 | 0,0048 | 75 |
| J3imm - J3T0 | -1,13 ± 0,27 | <0,001 | 81 |
| J4imm - J4T0 | -1,00 ± 0,27 | 0,0027 | 75 |
| J5imm - J5T0 | -1,13 ± 0,36 | 0,0074 | 75 |
| J6imm - J6T0 | -1,19 ± 0,33 | 0,0027 | 75 |
| J7imm -J7T0 | -1,19 ± 0,33 | 0,0027 | 69 |
| J8imm - J8T0 | -1,13 ± 0,36 | 0,0074 | 75 |
| J9imm - J9T0 | -0,94 ± 0,40 | 0,0371 | 63 |
| J10imm - J10T0 | -0,75 ± 0,34 | 0,0410 | 56 |
| J11imm - J11T0 | -0,56 ± 0,24 | 0,0339 | 56 |

La comparaison entre les différents temps (par rapport J1T0, avant application) se fait par une analyse statistique sur les rangs avec le test de Wilcoxon-Mann-Whitney. La significativité est atteinte lorsque p<0,05. esm : erreur standard à la moyenne.

Un effet immédiat significatif sur la réduction de la sensation d'inconfort perçu comme douloureux est démontré dès le premier jour et durant les 11 premiers jours d'application et ce chez une grande proportion des patients.

L'effet cumulatif est résumé dans le tableau 14 ci-dessous.

**Tableau 14**

| Cinétique | Score moyenné ± esm | Statistique Valeur p | % de sujets avec une amélioration |
|---|---|---|---|
| J3T0 - J1T0 | -1,25 ± 0,63 | 0,0469 | 38 |
| J4T0 - J1T0 | -1,56 ± 0,66 | 0,0039 | 56 |
| J5T0 - J1T0 | -2,00 ± 0,67 | 0,0020 | 63 |
| J6T0 - J1T0 | -2,31 ± 0,70 | 0,0020 | 63 |
| J7T0 - J1T0 | -2,56 ± 0,71 | 0,0027 | 69 |
| J8T0 - J1T0 | -3 00 ± 0,72 | <0,001 | 81 |
| J9T0 - J1T0 | -3,50 ± 0,78 | <0,001 | 81 |
| J10T0 - J1T0 | -3,88 ± 0,74 | <0,001 | 88 |
| J11T0 - J1T0 | -4,13 ± 0,71 | <0,001 | 88 |
| J12T0 -J1T0 | -4,69 ± 0,72 | <0,001 | 88 |
| J13T0 - J1T0 | -4,69 ± 0,72 | <0,001 | 94 |
| J14T0 - J1T0 | -4,94 ± 0,72 | <0,001 | 94 |
| J15T0 - J1T0 | -5,06 ± 0,69 | <0,001 | 94 |
| J16T0 - J1T0 | -5,19 ± 0,71 | <0,001 | 94 |
| J17T0 - J1T0 | -5,38 ± 0,73 | <0,001 | 94 |
| J18T0 - J1T0 | -5,50 ± 0,72 | <0,001 | 88 |
| J19T0 - J1T0 | -5,56 ± 0,70 | <0,001 | 94 |
| J20T0 - J1T0 | -5,75 ± 0,73 | <0,001 | 94 |
| J21T0 - J1T0 | -5,88 ± 0,72 | <0,001 | 94 |

La comparaison entre les différents temps (par rapport JT0, avant application) se fait par une analyse statistique sur les rangs avec le test de Wilcoxon-Mann-Whitney. La significativité est atteinte lorsque p<0,05. esm : erreur standard à la moyenne.

Un effet cumulatif statistiquement significatif sur la réduction de la sensation d'inconfort perçu comme douloureux est clairement démontré à partir du jour3 et ce pendant toute la durée du traitement.

En conclusion, dans cette étude de 16 patients la composition selon l'invention est extrêmement bien tolérée chez les patients atteints d'eczéma. Elle montre une efficacité apaisante immédiate et durable durant toute la durée du traitement. Un effet est également démontré sur la réduction de la sensation d'inconfort perçu comme douloureux.

## Revendications

1. Composition cosmétique ou dermatologique sous forme d'émulsion pour application topique, comprenant :
- une phase huileuse ; et
- une phase aqueuse comprenant un carbomère, un polysaccharide naturel et un copolymère acrylique ou méthacrylique réticulé modifié hydrophobiquement ;
ladite composition comprenant de 0,10 à 0,25%, de préférence de 0,15 à 0,20%, en poids de carbomère par rapport au poids total de la composition.

2. Composition cosmétique ou dermatologique pour application topique selon la revendication 1, **caractérisée en ce qu'**elle comprend 0,01 à 0,15%, notamment 0,05 à 0,10% en poids de polysaccharide naturel par rapport au poids total de la composition.

3. Composition cosmétique ou dermatologique pour application topique selon l'une des revendications 1 et 2, **caractérisée en ce qu'**elle comprend 0,10 à 0,25%, notamment 0,12 à 0,20% en poids de copolymère acrylique ou méthacrylique réticulé modifié hydrophobiquement par rapport au poids total de la composition.

4. Composition cosmétique ou dermatologique pour application topique selon l'une quelconque des revendications 1 à 3, **caractérisée en ce qu'**elle comprend en outre un extrait de plantule d'avoine.

5. Composition cosmétique ou dermatologique pour application topique selon l'une quelconque des revendications 1 à 4, **caractérisée en ce qu'**elle est formulée sous forme de spray.

6. Composition cosmétique ou dermatologique pour application topique selon la revendication 5, **caractérisée en ce que** le spray fonctionne sans gaz propulseurs.

7. Composition cosmétique ou dermatologique pour application topique selon l'une quelconque des revendications 1 à 6, pour son utilisation apaisante, calmante et/ou anti-irritante.

8. Composition dermatologique selon l'une quelconque des revendications 1 à 6, pour son utilisation dans le traitement de l'inflammation neurogène cutanée par application topique.

9. Composition dermatologique selon l'une quelconque des revendications 1 à 6, pour son utilisation dans le traitement des dermatoses inflammatoires choisies parmi la dermatite atopique, l'eczéma et le psoriasis, par application topique.

10. Composition dermatologique selon l'une quelconque des revendications 1 à 6, pour son utilisation dans le traitement des sensations de démangeaisons et/ou des douleurs cutanées par application topique.

11. Composition dermatologique pour son utilisation selon la revendication 10, **caractérisée par le fait que** les démangeaisons et/ou les douleurs cutanées sont dues à une piqûre, un coup ou à un acte médical ou chirurgical par application topique.

## Patentansprüche

1. Kosmetische oder dermatologische Zusammensetzung in Form einer Emulsion zur topischen Anwendung, umfassend:
- eine Ölphase; und
- eine wässrige Phase, die ein Carbomer, ein natürliches Polysaccharid und ein hydrophob modifiziertes vernetztes Acryl- oder Methacryl-Copolymer umfasst;
wobei die Zusammensetzung 0,10 bis 0,25 Gew.-%, vorzugsweise 0,15 bis 0,20 Gew.-% Carbomer, bezogen auf das Gesamtgewicht der Zusammensetzung, umfasst.

2. Kosmetische oder dermatologische Zusammensetzung zur topischen Anwendung nach Anspruch 1, **dadurch gekennzeichnet, dass** sie 0,01 bis 0,15 Gew.-%, insbesondere 0,05 bis 0,10 Gew.-% natürliches Polysaccharid, bezogen auf das Gesamtgewicht der Zusammensetzung, umfasst.

3. Kosmetische oder dermatologische Zusammensetzung zur topischen Anwendung nach einem der Ansprüche 1 und 2, **dadurch gekennzeichnet, dass** sie 0,10 bis 0,25 Gew.-%, insbesondere 0,12 bis 0,20 Gew.-% hydrophob modifiziertes vernetztes Acryl- oder Methacryl-Copolymer, bezogen auf das Gesamtgewicht der Zusammensetzung, umfasst.

4. Kosmetische oder dermatologische Zusammensetzung zur topischen Anwendung nach einem der Ansprüche 1 bis 3, **dadurch gekennzeichnet, dass** sie ferner einen Extrakt aus Haferkeimen umfasst.

5. Kosmetische oder dermatologische Zusammensetzung zur topischen Anwendung nach einem der Ansprüche 1 bis 4, **dadurch gekennzeichnet, dass** sie in Form eines Sprays formuliert ist.

6. Kosmetische oder dermatologische Zusammensetzung zur topischen Anwendung nach Anspruch 5, **dadurch gekennzeichnet, dass** das Spray ohne Treibgase funktioniert.

7. Kosmetische oder dermatologische Zusammensetzung zur topischen Anwendung nach einem der Ansprüche 1 bis 6 zu ihrer beruhigenden, lindernden und/oder reizhemmenden Verwendung.

8. Dermatologische Zusammensetzung nach einem der Ansprüche 1 bis 6 zu ihrer Verwendung bei der Behandlung der neurogenen Hautentzündung durch topische Anwendung.

9. Dermatologische Zusammensetzung nach einem der Ansprüche 1 bis 6 zu ihrer Verwendung bei der Behandlung von entzündlichen Hauterkrankungen, ausgewählt aus der atopischen Dermatitis, dem Ekzem und der Psoriasis, durch topische Anwendung.

10. Dermatologische Zusammensetzung nach einem der Ansprüche 1 bis 6 zu ihrer Verwendung bei der Behandlung von Juckreiz und/oder Hautschmerzen durch topische Anwendung.

11. Dermatologische Zusammensetzung zu ihrer Verwendung nach Anspruch 10, **dadurch gekennzeichnet, dass** der Juckreiz und/oder die Hautschmerzen auf einen Stich, einen Schlag oder eine medizinischen oder chirurgischen Eingriff durch topische Anwendung zurückzuführen sind.

## Claims

1. Cosmetic or dermatological composition in the form of an emulsion for topical application, comprising:
- an oil phase; and
- an aqueous phase comprising a carbomer, a natural polysaccharide and a hydrophobically modified crosslinked acrylic or methacrylic copolymer;
said composition comprising from 0.10 to 0.25%, preferably from 0.15 to 0.20%, by weight of carbomer relative to the total weight of the composition.

2. Cosmetic or dermatological composition for topical application according to claim 1, **characterized in that** it comprises 0.01 to 0.15%, in particular 0.05 to 0.10% by weight of natural polysaccharide relative to the total weight of the composition.

3. Cosmetic or dermatological composition for topical application according to one of claims 1 and 2, **characterized in that** it comprises 0.10 to 0.25%, in particular 0.12 to 0.20% by weight of hydrobophobically modified crosslinked acrylic or methacrylic copolymer relative to the total weight of the composition.

4. Cosmetic or dermatological composition for topical application according to any of claims 1 to 3, **characterized in that** it further comprises an oat seedling extract.

5. Cosmetic or dermatological composition for topical application according to any of claims 1 to 4, **characterized in that** it is formulated in spray form.

6. Cosmetic or dermatological composition for topical application according to claim 5, **characterized in that** the spray works without propellants.

7. Cosmetic or dermatological composition for topical application according to any of claims 1 to 6, for its soothing, calming and/or anti-irritant use.

8. Dermatological composition according to any of claims 1 to 6, for use in the treatment of neurogenic skin inflammation by topical application.

9. Dermatological composition according to any of claims 1 to 6, for use in the treatment of inflammatory dermatoses selected from atopic dermatitis, eczema and psoriasis, by topical application.

10. Dermatological composition according to any of claims 1 to 6, for use in the treatment of itching and/or skin pain by topical application.

11. Dermatological composition for use according to claim 10, **characterized in that** the itching and/or skin pain is caused by a sting, a blow or a medical or surgical procedure, for topical application.
